# EUROPEAN PATENT SPECIFICATION

(11) **EP 1 171 619 B1**
(45) Date of publication and mention of the grant of the patent: **15.06.2011**
(21) Application number: 00925970.6
(22) Date of filing: 13.04.2000
(51) Int. Cl.: C12N 15/82, C12N 15/54, A01H 5/00

(54) **Regulated expression of isopentenyl transferase in maize seeds**
Regulierte Expression der Isopentenyltransferase in Maissamen
Expression regulée de l'isopentényltransférase en semences de maïs

(30) Priority: 16.04.1999 US 129844 P
(43) Date of publication of application: 16.01.2002
(73) Proprietor: PIONEER HI-BRED INTERNATIONAL, INC., Johnston, IA 50131-1000 (US)
(72) Inventor: HABBEN, Jeffrey, E., Urbandale, IA 50322 (US); ZINSELMEIER, Christopher, Des Moines, IA 50310 (US); TOMES, Dwight, Van Meter, IA 50261 (US)
(74) Representative: Bentham, Andrew
(86) International application number: PCT/US2000/009943
(87) International publication number: WO 2000/063401

(56) References cited:
- WO-A-93/07272
- WO-A-96/29858
- WO-A-98/00545
- WO-A-99/06571
- VANTOI, T., ET AL.: "IMPROVED BIOMASS PRODUCTION AND ABIOTIC STRESS TOLERANCE OF TRANSGENEIC ARABIDOPSIS CONTAINING AN AUTOREGULATED CYTOKININ BIOSYNTHESIS GENE " PLANT AND ANIMAL GENOME VI CONFERENCE, SAN DIEGO, CA, JANUARY 17-21, 1999. HTTP://WWW.INTL-PAG.ORG/PAG/7/ABSTRACTS/PA G7501.HTML, January 1999 (1999-01), page 518 XP002146025
- ROECKEL PATRICIA ET AL: "Effects of seed-specific expression of a cytokinin biosynthetic gene on canola and tobacco phenotypes." TRANSGENIC RESEARCH, vol. 6, no. 2, 1997, pages 133-141, XP000938801 ISSN: 0962-8819 cited in the application
- MA QING-HU ET AL: "Seed-specific expression of the isopentenyl transferase gene (ipt) in transgenic tobacco." AUSTRALIAN JOURNAL OF PLANT PHYSIOLOGY, vol. 25, no. 1, 1998, pages 53-59, XP000938791 ISSN: 0310-7841
- MIROSLAV KAMINEK: "PROGRESS IN CYTOKININ RESEARCH" TRENDS IN BIOTECHNOLOGY,GB,ELSEVIER PUBLICATIONS, CAMBRIDGE, vol. 10, no. 5, May 1992 (1992-05), pages 159-164, XP000272384 ISSN: 0167-7799
- ROECKEL P ET AL: "Phenotypic alterations and component analysis of seed yield in transgenic Brassica napus plants expressing the tzs gene." PHYSIOLOGIA PLANTARUM, vol. 102, no. 2, February 1998 (1998-02), pages 243-249, XP000938784 ISSN: 0031-9317
- HEINRICH, G.M. ET AL.: "Mechanisms of yield stability in sorghum" CROP SCIENCE, vol. 25, no. 6, December 1985 (1985-12), pages 1109-1113,

## Description

### Field of the Invention:

This invention relates generally to the field of plant molecular biology. More specifically, this invention relates to methods and reagents for the temporal and/or spatial expression of genes that affect metabolically effective levels of cytokinins in plant seeds, as well as in the maternal tissue from which such seeds arise, including developing ears, female inflorescences, ovaries, female florets, aleurone, pedicel, and pedicel-forming regions.

### Background of the Invention:

Cytokinins have been demonstrated to play a fundamental role in establishing seed size, decreasing tip kernel abortion and increasing seed set during unfavorable environmental conditions. The first naturally occurring cytokinin was purified in 1963 (Letham, D.S., Life Sci. 8:569-573 (1963)) from immature kernels of *Zea mays* and identified as 6-(4-hydroxy-3-methylbut-trans-2-enylamino) purine, more commonly known today as zeatin. In the main all naturally occurring cytokinins appear to be purine derivatives with a branched 5-carbon N⁶ substitutent. (See: McGaw, B.A., In: Plant Hormones and their Role in Plant Growth and Development, ed. P.J. Davies, Martinus Nijhoff Publ., Boston, 1987, Chap B3, Pgs. 76-93). While some 25 different naturally occurring cytokinins have been identified, those regarded as particularly active are N⁶ (Δ²-isopentenyl) adenosine (iP), zeatin (Z), diHZ, benzyladenine (BAP) and their 9-ribosyl (and in the case of Z and diHZ, their O-glucosyl) derivatives. However, such activity is markedly reduced in the 7- and 9-glucosyl and 9-alanyl conjugates. These latter compounds may be reflective of deactivation or control mechanisms.

The metabolism of cytokinins in plants is complex. Multi-step biochemical pathways are known for the biosynthesis and degradation of cytokinins. At least two major routes of cytokinin biosynthesis are recognized. The first involves transfer RNA (tRNA) as an intermediate. The second involves *de novo* (direct) biosynthesis. In the first case, tRNAs are known to contain a variety of hypermodified bases (among them are certain cytokinins). These modifications are known to occur at the tRNA polymer level as a post-transcriptional modification. The branched 5-carbon N⁶ substituent is derived from mevalonic acid pyrophosphate, which undergoes decarboxylation, dehydration, and isomerization to yield Δ²-isopentenyl pyrophosphate (iPP). The latter condenses with the relevant adenosine residue in the tRNA. Further modifications are then possible. Ultimately the tRNAs are hydrolyzed to their component bases, thereby forming a pool of available free cytokinins.

Alternately, enzymes have been discovered that catalyze the formation of cytokinins *de novo*, i. e., without a tRNA intermediate. The ipt gene utilized in the practice of this invention is one such gene. The formation of free cytokinins is presumed to begin with [9R5'P] iP. This compound is rapidly and stereospecifically hydroxylated to give the zeatin derivatives from which any number of further metabolic events may ensue. Such events include but are not limited to (1) conjugation, incorporating ribosides, ribotides, glucosides, and amino acids; (2) hydrolysis; (3) reduction; and (4) oxidation. While each enzyme in these pathways is a candidate as an effector of cytokinin levels, enzymes associated with rate-limiting steps have particular utility in the practice of this invention.

One such enzyme is isopentenyl transferase (ipt). An isolated gene encoding ipt was described by van Larebeke et al., (Nature 252:169-170(1974)). Smigocki et al. (Proc. Nat'l. Acad. Sci. (USA) 85:5131-5135(1988)), employing the ipt gene from A. tumefaciens operably linked to either the 35S or NOS promoter, showed a generalized effect on shoot organogenesis and zeatin levels. Such unregulated production of cytokinins can result in unwanted pleiotropic effects. For example, with the constructions identified above, Smigocki *et al.* (supra) reported that typically complete inhibition of root formation was observed.

Attempts followed to express the ipt gene in a more controlled fashion. Medford et al. (The Plant Cell 1:403-413(1989)) reported placing the ipt gene under the control of a heat-inducible promoter and expressing same in transgenic rooted tobacco plants. While the levels of cytokinin rose dramatically following heat treatment, the promoters were not wholly satisfactory because the plants exhibited phenotypes associated with excess cytokinin levels even in the absence of thermal induction. See also: Schumulling, T. et al. (FEBS Letters 249(2):401-406(1989)). A more regulated response was reported in PCT Patent Application Publication No. WO91/01323, 7 February 1991, and PCT Patent Application Publication No. WO93/07272, 15 April 1993, both assigned on their face to Calgene, in which the ipt gene was fused to the chalcone synthase (chs) promoter from Antirrhinum majus and expressed in potato.

Additional ipt gene/promoter constructions have been reported. Smigocki et al., in U.S. Patent 5,496,732, disclosed a gene construct capable of conferring enhanced insect resistance comprising a wound-inducible promoter fused to an ipt gene. Houck et al., in U. S. Patents 4,943,674 and 5,177,307, disclosed several promoters (2AII, Z130 and Z70) coupled with genes encoding enzymes in the cytokinin metabolic pathway, in particular ipt for expression of such enzymes in tomato fruit. Amasino et al., in PCT Patent Application Publication WO96/29858 disclosed two senescence gene promoters operably linked to an ipt gene to inhibit leaf senescence in tobacco. See also: Gan, S. et al., (Science 270:1986-1988 (1995)). Roeckel, P. et a/., (Transgenic Res. 6(2):133-141 (1997)) transformed canola and tobacco with an ipt gene under the control of a 2S albumin promoter from Agrobacterium. Increase in branching of inflorescences was noted, but increases in seed yield and seed weight were not observed.

There still exists a need for the controlled expression, both temporally and spatially, of cytokinin metabolic genes in plant seed and in those maternal tissues in which seed development takes place. This invention addresses this need by providing several useful genetic constructs and methods to modulate effective levels of cytokinin in plant seeds, developing plant seeds, and related maternal tissues. These related maternal tissues would include such tissues as the female floret, the ovary, aleurone, pedicel, and the pedicel-forming region. The maternal tissues are also referred to as "grain initials" or "seed initials".

This invention differs from the foregoing approaches in that it provides methods that allow the skilled artisan, by the application of, *inter alia*, transgenic methodologies to influence the metabolic flux in respect to the cytokinin metabolic pathway in seed. This influence is anabolic, by which is meant the influence may act to increase the flow resulting from the biosynthesis of cytokinin. A combination of this approach with a decrease in the degradation (i.e., catabolism of) cytokinins is also contemplated by this invention.

### Summary of the Invention

The invention provides a method for improving yield in *Zea mays* plants comprising:
- stably introducing into cells of said plants a genetic construct capable of preferentially expressing a cytokinin modulating gene which encodes a cytokinin biosynthetic enzyme in one or more tissues of developing seed and related maternal tissues within the range of from 14 days before pollination to 25 days after pollination; and
- regenerating and recovering plants from said cells;
wherein said cytokinin modulating gene is isopentenyl transferase and wherein said genetic construct comprises an Itp2 promoter directing temporal and/or
spatial gene expression in plant seed operably linked to the cytokinin modulating gene.

The invention further provides use of a stably introduced genetic construct capable of preferentially expressing a cytokinin modulating gene which encodes a cytokinin biosynthetic enzyme in one or more tissues of developing seed and related maternal tissue within the range of from 14 days before pollination to 25 days after pollination, to improve yield in a Zea mays plant, wherein said cytokinin modulating gene is isopentenyl transferase and wherein said genetic construct comprises an ltp2 promoter directing temporal and/or spatial gene expression in plant seed operably linked to the cytokinin modulating gene.

The invention further provides a fertile transgenic *Zea mays* plant comprising a genetic construct stably integrated into the genome thereof, wherein said construct comprises an Itp2 promoter operably linked to the cytokinin-modulating gene isopentenyl transferase, wherein said promoter directs temporal and/or spatial expression in plant seed.

The invention further provides seeds of the transgenic plant of the invention, wherein said seeds contain the genetic construct comprising the Itp2 promoter operably linked to the cytokinin-modulating gene isopentenyl transferase.

It is therefore an object of the present invention to provide plants, particularly transgenic com, which have enhanced levels of cytokinins in the seed without corresponding detrimental effects.

It is a further object of the present invention to provide transgenic plant lines with dominant, heritable phenotypes which are useful in breeding programs designed to produce commercial products with improved seed size, decreased tip kernel abortion and increased seed set during unfavorable environmental conditions.

In accordance with this aspect of the invention there are disclosed isolated nucleic acid molecules encoding cytokinin metabolic enzymes, mRNAs, cDNAs, genomic DNAs and, in further embodiments of this aspect of the invention, biologically useful variants, analogs or derivatives thereof, or fragments thereof, including fragments of the variants, analogs and derivatives.

Also disclosed are naturally occurring allelic variants of the nucleic acid molecules in the sequences provided which encode cytokinin metabolic enzymes.

Described herein are polypeptides which comprise cytokinin metabolic enzymes as well as biologically or diagnostically useful fragments thereof, as well as variants, derivatives and analogs of the foregoing and fragments thereof.

Also described herein are cytokinin metabolic polypeptides, particularly ipt and cytokinin oxidase, that may be employed for modulation of cytokinin levels in seed.

In accordance with another object of the invention there are provided processes and methods that utilize the aforementioned polypeptides and polynucleotides for research, biological and agricultural purposes.

It is therefore an object of the present invention to provide plants, particularly transgenic com, which have enhanced levels of cytokinins in the seed, the developing seed, and the maternal tissues associated with seed development. These levels act as a metabolic buffer to ameliorate the effects of transient stresses, particularly during the lag phase of seed development, to thus improve com stress tolerance and yield stability.

It is a further object of the present invention to provide transgenic com which has enhanced levels of cytokinins in the seed to provide commercial products with improved seed size, decreased tip kernel abortion and increased seed set during unfavorable environmental conditions.

It is a further object of this invention to provide a method for producing fertile, transgenic plants capable of the regulated expression of a cytokinin modulating gene in developing seeds, comprising introducing into plant host cells a genetic construct capable of preferential temporal and/or spatial expression of a cytokinin-modulating gene in developing seed and the maternal tissues associated with seed development, under conditions sufficient for the stable integration of the construct into the genome of said cells, and regenerating and recovering said fertile transgenic plants.

It is a further object of this invention to provide a fertile transgenic plant comprising a genetic construct stably integrated into the genome thereof, said construct capable of the temporal and/or spatial modulation of cytokinin levels in developing seed of said plant.

Also described herein is an isolated recombinant DNA comprising a genetic construct that comprises a promoter directing temporal and/or spatial gene expression in plant seed operatively linked to a cytokinin modulating gene.

It is a further object of this invention to provide a method for improving and stability in plants in need thereof, comprising stably introducing into cells of said plants a genetic construct capable of preferentially expressing cytokinin modulating genes during the lag phase of plant seed development, and regenerating and recovering plants from said cells.

### Brief Description of the Drawings:

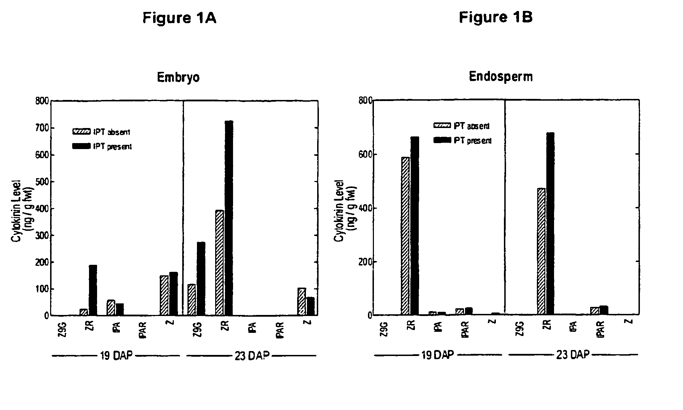

Figure 1A-Embryo: This Figure shows that embryo-preferred overexpression of ipt increases embryo cytokinin levels, particularly ZR and Z9G (range of 2 to 8-fold difference). In contrast, Z levels are unchanged and IPAR is not detectable at either developmental stage. Abbreviations: Z=zeatin, ZR (or [9R]Z)=zeatin riboside, Z9G (or [9G]Z)=zeatin-9-glucoside, IPA or [9R]iP=isopentenyladenosine, IPAR (or [9R-5'P]iP)=isopentenyladenosine-5'-monophosphate, and DAP=Days After Pollination.
Figure 1B-Endosperm: This Figure shows that embryo-preferred ipt overexpression altered endosperm cytokinin levels but less than those in the embryo (range of only 10 to 30% difference). Abbreviations used as in Figure 1A.

### Glossary:

The following illustrative explanations are provided to facilitate understanding of certain terms used frequently herein, particularly in the Examples. The explanations are provided as a convenience and are not limitative of the invention.

CYTOKININ METABOLIC ENZYME-BINDING MOLECULE, as used herein, refers to molecules or ions which bind or interact specifically with cytokinin metabolic enzyme polypeptides or polynucleotides described herein, including, for example enzyme substrates, cell membrane components and classical receptors. Binding between polypeptides described herein and such molecules, including binding or interaction molecules, may be exclusive to said polypeptides, which is preferred, or it may be highly specific for said polypeptides, which is also preferred, or it may be highly specific to a group of proteins that includes said polypeptides, which is preferred, or it may be specific to several groups of proteins at least one of which includes a polypeptide described herein. Binding molecules also include antibodies and antibody-derived reagents that bind specifically to said polypeptides.

CYTOKININ RESPONSIVE COMPONENT, as used herein, generally means a cellular constituent that binds to or otherwise interacts with a cytokinin resulting in the transmission of an intra- or inter- cellular signal and eliciting one or more cellular responses to the presence or absence or fluctuation in the levels of cytokinins.

DEVELOPING PLANT SEEDS, as used herein, generally means the maternal plant tissues which after pollination are capable of giving rise to a plant seed. This maternal plant tissue includes such tissue as female florets, ovaries, aleurone, pedicel, and pedicel-forming region.

GENETIC ELEMENT, as used herein, generally means a polynucleotide comprising a region that encodes a polypeptide or a polynucleotide region that regulates replication, transcription or translation or other processes important to expression of the polypeptide in a host cell, or a polynucleotide comprising both a region that encodes a polypeptide and a region operably linked thereto that regulates expression. Genetic elements may be comprised within a vector that replicates as an episomal element; that is, as a molecule physically independent of the host cell genome. They may be comprised within plasmids. Genetic elements also may be comprised within a host cell genome; not in their natural state but, rather, following manipulation such as isolation, cloning and introduction into a host cell in the form of purified DNA or in a vector, among others.

GERMPLASM, as used herein, means a set of genetic entities, which may be used in a conventional breeding program to develop new plant varieties.

HIGH CYTOKININ TRANSGENIC, as used herein, means an entity, which, as a result of recombinant genetic manipulation, produces seed with a heritable increase in cytokinin and/or decrease in auxin.

HOST CELL, as used herein, is a cell which has been transformed or transfected, or is capable of transformation or transfection by an exogenous polynucleotide sequence. Exogenous polynucleotide sequence is defined to mean a sequence not naturally in the cell. This includes transformation to incorporate additional copies of an endogenous polynucleotide.

IDENTITY and SIMILARITY, as used herein, and as known in the art, are relationships between two polypeptide sequences or two polynucleotide sequences, as determined by comparing the sequences. In the art, identity also means the degree of sequence relatedness between two polypeptide or two polynucleotide sequences as determined by the match between two strings of such sequences. Both identity and similarity can be readily calculated (Computational Molecular Biology, Lesk, A.M., ed., Oxford University Press. New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). Methods commonly employed to determine identity or similarity between two sequences include, but are not limited to those disclosed in Carillo, H., and Lipman, D., SIAM J. Applied Math., 48:1073 (1988). Preferred methods to determine identity are designed to give the largest match between the two sequences tested. Methods to determine identity and similarity are codified in computer programs. Typical computer program methods to determine identity and similarity between two sequences include: GCG program package (Devereux, J., et al., Nucleic Acids Research 12(1):387 (1984)), BLASTP, BLASTN, FASTA and TFASTA (Atschul, S.F. et al., J. Mol. Biol. 215:403 (1990)).

For purposes of defining the present invention, the Gap program is used. The algorithm used for the Gap program is that of Needleman and Wunsch (J. Mol. Biol. 48:443-453 [1970]). The parameters used are as follows: for nucleotide comparisons the gap creation penalty = 50, gap extension penalty = 3; for amino acid comparisons the gap creation penalty = 12, the gap extension penalty = 4.

ISOLATED, as used herein, means altered "by the hand of man" from its natural state; i.e., that, if it occurs in nature, it has been changed or removed from its original environment, or both. For example, a naturally occurring polynucleotide or a polypeptide naturally present in a living organism in its natural state is not "isolated," but the same polynucleotide or polypeptide separated from the coexisting materials of its natural state is "isolated", as the term is employed herein. For example, with respect to polynucleotides, the term isolated means that it is separated from the chromosome and cell in which it naturally occurs. As part of or following isolation, such polynucleotides can be joined to other polynucleotides, such as DNAs, for mutagenesis, to form fusion proteins, and for propagation or expression in a host, for instance. The isolated polynucleotides, alone or joined to other polynucleotides such as vectors, can be introduced into host cells, in culture or in whole organisms. Introduced into host cells in culture or in whole organisms, such DNAs still would be isolated, as the term is used herein, because they would not be in their naturally occurring form or environment. Similarly, the polynucleotides and polypeptides may occur in a composition, such as media formulations, solutions for introduction of polynucleotides or polypeptides, for example, into cells, compositions or solutions for chemical or enzymatic reactions, for instance, which are not naturally occurring compositions, and, therein remain isolated polynucleotides or polypeptides within the meaning of that term as it is employed herein.

LIGATION, as used herein, refers to the process of forming phosphodiester bonds between two or more polynucleotides, which most often are double stranded DNAs. Techniques for ligation are well known to the art and protocols for ligation are described in standard laboratory manuals and references, such as, for instance, Sambrook et al., MOLECULAR CLONING, A LABORATORY MANUAL, 2nd Ed.; Cold Spring Harbor Laboratory Press, Cold Spring Harbor, New York (1989) and Maniatis *et al.,* pg. 146, as cited below.

OLIGONUCLEOTIDE(S), as used herein, refers to short polynucleotides. Often the term refers to single-stranded deoxyribonucleotides, but it can refer as well to single- or double-stranded ribonucleotides, RNA:DNA hybrids and double-stranded DNAs, among others. Oligonucleotides, such as single-stranded DNA probe oligonucleotides, often are synthesized by chemical methods, such as those implemented on automated oligonucleotide synthesizers. However, oligonucleotides can be made by a variety of other methods, including *in vitro* recombinant DNA-mediated techniques and by expression of DNAs in cells and organisms. Initially, chemically synthesized DNAs typically are obtained without a 5' phosphate. The 5' ends of such oligonucleotides are not substrates for phosphodiester bond formation by ligation reactions that employ DNA ligases typically used to form recombinant DNA molecules. Where ligation of such oligonucleotides is desired, a phosphate can be added by standard techniques, such as those that employ a kinase and ATP. The 3' end of a chemically synthesized oligonucleotide generally has a free hydroxyl group and, in the presence of a ligase, such as T4 DNA ligase, readily will form a phosphodiester bond with a 5' phosphate of another polynucleotide, such as another oligonucleotide. As is well known, this reaction can be prevented selectively, where desired, by removing the 5' phosphates of the other polynucleotide(s) prior to ligation.

OPERABLY LINKED, as used herein, includes reference to a functional linkage between a promoter and a second sequence, wherein the promoter sequence initiates and mediates transcription of the DNA sequence corresponding to the second sequence. Generally, operably linked means that the nucleic acid sequences being linked are contiguous and, where necessary to join two protein coding regions, contiguous and in the same reading frame.

PLANT, as used herein, includes reference to whole plants, plant parts or organs (e.g., leaves, stems, roots, etc.), plant cells, seeds and progeny of same. Plant cell, as used herein, further includes, without limitation, cells obtained from or found in: seeds, suspension cultures, embryos, meristematic regions, callus tissue, leaves, roots, shoots, gametophytes, sporophytes, pollen, and microspores. Plant cells can also be understood to include modified cells, such as protoplasts, obtained from the aforementioned tissues. The plant which can be used in the methods of the invention is *Zea mays.*

PLASMIDS, as used herein, generally are designated herein by a lower case p preceded and/or followed by capital letters and/or numbers, in accordance with standard naming conventions that are familiar to those of skill in the art. Starting plasmids disclosed herein are either commercially available, publicly available, or can be constructed from available plasmids by routine application of well known, published procedures. Many plasmids and other cloning and expression vectors that can be used in accordance with the methods of the present invention are well known and readily available to those of skill in the art. Moreover, those of skill readily may construct any number of other plasmids suitable for use in the invention. The properties, construction and use of such plasmids, as well as other vectors, for use in the present invention will be readily apparent from the present disclosure to those of skill.

POLYNUCLEOTIDE(S), as used herein, generally refers to any polyribonucleotide or polydeoxribonucleotide, which may be unmodified RNA or DNA or modified RNA or DNA. Thus, for instance, polynucleotides as used herein refers to, among others, single-and double-stranded DNA, DNA that is a mixture of single- and double-stranded regions or single-, double- and triple-stranded regions, single- and double-stranded RNA, and RNA that is a mixture of single- and double-stranded regions, hybrid molecules comprising DNA and RNA that may be single-stranded or, more typically, double-stranded, or triple-stranded, or a mixture of single- and double-stranded regions. In addition, polynucleotide as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. As used herein, the term polynucleotide includes DNAs or RNAs as described above that contain one or more modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritylated bases, to name just two examples, are polynucleotides as the term is used herein. It will be appreciated that a great variety of modifications have been made to DNA and RNA that serve many useful purposes known to those of skill in the art. The term polynucleotide as it is employed herein embraces such chemically-, enzymatically- or metabolically-modified forms of polynucleotides, as well as the chemical forms of DNA and RNA characteristic of viruses and cells, including *inter alia*, simple and complex cells.

POLYPEPTIDES, as used herein, includes all polypeptides as described below. The basic structure of polypeptides is well known and has been described in innumerable textbooks and other publications in the art. In this context, the term is used herein to refer to any peptide or protein comprising two or more amino acids joined to each other in a linear chain by peptide bonds. As used herein, the term refers to both short chains, which also commonly are referred to in the art as peptides, oligopeptides and oligomers, for example, and to longer chains, which generally are referred to in the art as proteins, of which there are many types. It will be appreciated that polypeptides often contain amino acids other than the 20 amino acids commonly referred to as the 20 naturally occurring amino acids, and that many amino acids, including the terminal amino acids, may be modified in a given polypeptide, either by natural processes, such as processing and other post-translational modifications, but also by chemical modification techniques which are well known to the art. Even the common modifications that occur naturally in polypeptides are too numerous to list exhaustively here, but they are well described in basic texts and in more detailed monographs, as well as in a voluminous research literature, and they are well known to those of skill in the art. Among the known modifications which may be present in polypeptides of the present are, to name an illustrative few, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphatidylinositol, cross-linking, cyclization, disulfide bond formation, demethylation, formation of covalent cross-links, formation of cystine, formation of pyroglutamate, formylation, gamma-carboxytation, glycosylation, GPI anchor formation, hydroxylation, iodination, methylation, myristoylation, oxidation, proteolytic processing, phosphorytation, prenylation, racemization, selenoylation, sulfation, transfer-RNA mediated addition of amino acids to proteins such as arginylation, and ubiquitination. Such modifications are well known to those of skill and have been described in great detail in the scientific literature. Several particularty common modifications, glycosylation, lipid attachment, sulfation, gamma-carboxylation of glutamic acid residues, hydroxylation and ADP-ribosylation, for instance, are described in most basic texts, such as, for instance PROTEINS - STRUCTURE AND MOLECULAR PROPERTIES, 2nd Ed., T. E. Creighton, W. H. Freeman and Company, New York (1993). Many detailed reviews are available on this subject, such as, for example, those provided by Wold, F., Posttranslational Protein Modifications: Perspectives and Prospects, pgs. 1-12 in POSTTRANSLATlONAL COVALENT MODIFICATION OF PROTEINS, B. C. Johnson, Ed., Academic Press, New York (1983); Seifter et a/., Meth. Enzymol. 182:626-646 (1990) and Rattan et al., Protein Synthesis: Posttranslational Modifications and Aging, Ann. N.Y. Acad. Sci. 663:48-62 (1992). It will be appreciated, as is well known and as noted above, that polypeptides are not always entirely linear. For instance, polypeptides may be branched as a result of ubiquitination, and they may be circular, with or without branching, generally as a result of posttranslation events, including natural processing events and events brought about by human manipulation which do not occur naturally. Circular, branched and branched circular polypeptides may be synthesized by non-translation natural process and by entirely synthetic methods, as well. Modifications can occur anywhere in a polypeptide, including the peptide backbone, the amino acid side-chains and the amino or carboxyl termini. In fact, blockage of the amino or carboxyl group in a polypeptide, or both, by a covalent modification, is common in naturally occurring and synthetic polypeptide and such modifications may be present in polypeptides of the present invention, as well. For instance, the amino terminal residue of polypeptides made in *E. coli* or other cells, prior to proteolytic processing, almost invariably will be N-formylmethionine. During post-translational modification of the peptide, a methionine residue at the NH₂-terminus may be deleted. Accordingly, this invention contemplates the use of both the methionine-containing and the methionine-less amino terminal variants of the protein described herein. The modifications that occur in a polypeptide often will be a function of how it is made. For polypeptides made by expressing a cloned gene in a host, for instance, the nature and extent of the modifications in large part will be determined by the host cell post-translational modification capacity and the modification signals present in the polypeptide amino acid sequence. For instance, as is well known, glycosylation often does not occur in bacterial hosts such as, for example, *E. coli*. Accordingly, when glycosylation is desired, a polypeptide should be expressed in a glycosylating host, generally an eukaryotic cell. Similar considerations apply to other modifications. It will be appreciated that the same type of modification may be present in the same or varying degree at several sites in a given polypeptide. Also, a given polypeptide may contain many types of modifications. In general, as used herein, the term polypeptide encompasses all such modifications, particularly those that are present in polypeptides synthesized by expressing a polynucleotide in a host cell.

PROMOTER, as used herein, includes reference to a region of DNA upstream from the start of transcription and involved in recognition and binding of RNA polymerase and other proteins to initiate transcription. A "plant promoter" is a promoter capable of initiating transcription in plant cells. Exemplary plant promoters include, but are not limited to, those that are obtained from plants, plant viruses, and bacteria which comprise genes expressed in plant cells, such as *Agrobacterium* or *Rhizobium*. Examples of promoters under developmental control include promoters that preferentially initiate transcription in certain tissues, such as leaves, roots, or seeds or spatially in regions such as endosperm or embryos. Such promoters are referred to as "tissue preferred". Promoters that initiate transcription only in certain tissue are referred to as "tissue specific". A temporally regulated promoter drives expression at particular times, such as between 0-25 days after pollination. A "cell type" specific promoter primarily drives expression in certain cell types in one or more organs, for example, vascular cells in roots or leaves. An "inducible" promoter is a promoter which is under environmental control and may be inducible or derepressible. Examples of environmental conditions that may effect transcription by inducible promoters include anaerobic conditions or the presence of light. Tissue specific, tissue preferred, cell type specific, and inducible promoters constitute the class of "non-constitutive" promoters. A "constitutive" promoter is a promoter which is active under most environmental conditions.

RELATED MATERNAL TISSUE, as used herein, includes maternal plant tissues, such as female florets, ovaries, aleurone, pedicel, and pedicel-forming region, either pre-pollination or upon pollination. Pre-pollination seed tissues can also be referred to as "grain initials" or "seed initials".

TRANSFORMATION, as used herein, is the process by which a cell is "transformed" by exogenous DNA when such exogenous DNA has been introduced inside the cell membrane. Exogenous DNA may or may not be integrated (covalently linked) into chromosomal DNA making up the genome of the cell. In prokaryotes and yeasts, for example, the exogenous DNA may be maintained on an episomal element, such as a plasmid. With respect to higher eukaryotic cells, a stably transformed or transfected cell is one in which the exogenous DNA has become integrated into the chromosome so that it is inherited by daughter cells through chromosome replication. This stability is demonstrated by the ability of the eukaryotic cell to establish cell lines or clones comprised of a population of daughter cells containing the exogenous DNA.

VARIANT(S), as used herein, of polynucleotides or polypeptides, as the term is used herein, are polynucleotides or polypeptides that differ from a reference polynucleotide or polypeptide, respectively. Variants in this sense are described below and elsewhere in the present disclosure in greater detail. With reference to polynucleotides, generally, differences are limited such that the nucleotide sequences of the reference and the variant are closely similar overall and, in many regions, identical. As noted below, changes in the nucleotide sequence of the variant may be silent. That is, they may not after the amino adds encoded by the polynucleotide. Where alterations are limited to silent changes of this type, a variant will encode a polypeptide with the same amino add sequence as the reference. Also as noted below, changes in the nucleotide sequence of the variant may after the amino add sequence of a polypeptide encoded by the reference polynucleotide. Such nucleotide changes may result in amino add substitutions, additions, deletions, fusions and truncations in the polypeptide encoded by the reference sequence, as discussed below. With reference to polypeptides generally, differences are limited so that the sequences of the reference and the variant are closely similar overall and, in many regions, identical. A variant and reference polypeptide may differ in amino add sequence by one or more substitutions, additions, deletions, fusions and truncations, which may be present in any combination.

### Detailed Description of the Invention:

This invention may use to genetic constructs useful for the temporal and/or spatial expression of cytokinin genes in seed. The invention may also use associated polynucleotides and polypeptides; variants and derivatives of these polynucleotides and polypeptides. Also described herein are processes for making these polynucleotides and these polypeptides, and their variants and derivatives; and uses of these polynucleotides, polypeptides, variants, derivatives, agonists and antagonists. In particular, in these and in other regards, the invention relates to polynucleotides and polypeptides of the cytokinin metabolic pathway, specifically the enzyme ipt and the gene encoding same.

As mentioned above, described herein are the reagents necessary for the development of transgenic plants characterized by enhanced levels of cytokinin. As used herein, the phrase "enhanced levels of cytokinin" is a relative one and refers to the levels of cytokinin in a control plant without the cytokinin-affecting transgene as compared to a plant with such a functioning transgene. The relative levels may also be measured employing only the transgenic plant but measured in the presence and absence of expression of the subject transgene. Accordingly, any structural gene, the regulated expression of which has the effect of enhancing the effective levels of cytokinin in plants, particularly seeds, is useful for the practice of this invention. The ipt gene which directs the expression of proteins that act to increase the biosynthesis of cytokinin is used for the practice of this invention. The effective levels of cytokinins may be additionally reduced by cytokinin-binding molecules forming inactive or less-active conjugates. Thus gene products that act to release cytokinins from such conjugates (e.g., the product of the RolC gene or β-glucosidase) are also useful. In addition to genes that affect the absolute levels of cytokinin, genes that affect the ratio of cytokinin to auxin are also useful. Auxin-lowering genes such as iaa-1 and gene-5 may also be useful.

As mentioned above, the present invention relates to methods using novel constructions of cytokinin metabolic polypeptides and polynucleotides encoding same, among other things, as described in greater detail below. The polypeptide useful for the practice of this invention is ipt. The nucleic acids and fragments thereof encoding the above-mentioned enzyme are useful to generate enzyme-producing transgenics. For example, a single gene or gene fragment (or combinations of several genes) may be incorporated into an appropriate expression cassette and transformed into com along with an appropriate selectable marker (such as the BAR and PAT genes).

In certain situations it may be preferable to silence additionally certain genes, such as the cytokinin oxidase. Relevant literature describing the application of homology-dependent gene silencing include: Jorgensen, Trends Biotechnol. 8 (12):340-344 (1990); Flavell, Proc. Nat'l. Acad. Sci. (USA) 91:3490-3496 (1994); Finnegan et al., Bio/Technology 12: 883-888 (1994); Neuhuber et al., Mol. Gen. Genet. 244:230-241 (1994). Alternatively, another approach to gene silencing can be with the use of antisense technology (Rothstein et al. in Plant Mol. Cell. Biol. 6:221-246 (1989).

### Polynucleotides

Use of the isolated polynucleotide encoding ipt (isopentenyl transferase), as provided at Molecular and General Genetics 216:388-394 (1989), and its deduced amino acid sequence, are also contemplated by this invention.

Using the information provided herein, such as the polynucleotide sequences set out below, a polynucleotide for use of the present invention encoding cytokinin metabolic enzyme polypeptides may be obtained using standard cloning and screening procedures. To obtain the polynucleotide encoding the protein using the DNA sequences given below, oligonucleotide primers can be synthesized that are complementary to the known polynucleotide sequence. These primers can then be used in PCR to amplify the polynucleotide from template derived from mRNA or genomic DNA isolated from the desired source material. The resulting amplified products can then be cloned into commercially available cloning vectors, such as the TA series of vectors from InVitrogen. By sequencing the individual clones thus identified with sequencing primers designed from the original sequence, it is then possible to extend the sequence in both directions to determine the full gene sequence. Such sequencing is performed using denatured double stranded DNA prepared from a plasmid done. Suitable techniques are described by Maniatis, T., Fritsch, E.F. and Sambrook, J. in MOLECULAR CLONING, A Laboratory Manual (2nd edition 1989 Cold Spring Harbor Laboratory. See Sequencing Denatured Double-Stranded DNAS Templates 13.70

### Isolation of ipt gene:

The isopentenyl transferases (ipts) for use in the present invention may be obtained from the following sources: *Agrobacterium*, *Psuedomonas savastano*, *Rhodococcus* and *Erwinia*. The complete sequence of an ipt gene is provided in Strabala, T.J., et al., Isolation and characterization of an ipt gene from the Ti plasmid Bo542, Mol. Gen. Genet. 216, 388-94 (1989). A copy of such gene can be prepared synthetically employing DNA synthesis protocols well known to those skilled in the art of gene synthesis. Alternatively, a copy of the gene may be isolated directly from an ipt-gene-harboring organism by PCR cloning. Briefly, PCR primers preferably containing convenient restriction endonuclease sites are constructed. Two useful primers are shown below:
SEQ ID NO: 3 (Upper primer with Bam HI site)
   5'caucaucaucau**ggatc**caccaatggatctacgtctaattttcggtccaac 3'
SEQ ID NO:4 (Lower primer with Hpal site)
   5'cuacuacuacua**gttaac**tcacattcgaaatggtggtccttc 3'

The introduced restriction sites are bolded. The portion of the primer that binds to the template extends from nucleotides 22 and 19 to the 3' terminus, respectively. A BamHI site "ggatcc"(bolded) and a Kozak consensus sequence were introduced before the start codon and a Hpal site "gttaac" (also bolded) was introduced after the stop. Following is a schematic showing how the primers attach to the published sequence.

The *Agrobacterium tumefaciens* strain carrying the tumor-inducing plasmid pTi Bo542 was obtained (*Se*e Guyon, P., et al., Agropine in null-type crown gall tumors: Evidence for generality of the opine concept, Proceedings of the National Academy of Sciences (U.S.) 77(5): 2693-97 (1980); Chilton, W.S., et al. Absolute stereochemistry of leucinopine, a crown gall opine. Phytochemistry (Oxford) 24(2): 221-24 (1985); Strabala, T.J., et al., Isolation and characterization of an ipt gene from the Ti plasmid Bo542, Molecular & General Genetics 216: 388-94 (1989)) and live bacteria were used for the PCR template. Standard PCR conditions were used. An example of such conditions follows: Volume per reaction of 100 µL, with 0.5µL of 10 ng/µL target plasmid, 0.05 Unit/µL Taq Polymerase, 0.5 µM each of primers, 0.8 mM dNTP's 1X Buffer in a thin walled tube. Mix reagents, keep on ice. Add target plasmid to tube and then add the 100µL of reaction mix to each tube. Pre-incubate in a thermocycler at 95 °C for 3 minutes. Then cycle five times at 95 °C for 35 seconds, 55 °C for 1 minute, and 72 °C for 1 minute. Follow with 30 cycles at 95 °C for 35 seconds, 65 °C for 1 minute, and 72 °C for 1 minute. Finalize reaction by dwelling for 10 minutes at 72 °C and allowing to soak at 6 °C. PCR product was then cloned into DH5α cells using a kit made by Life Technologies according to manufacturer's instructions. DNA was extracted from putative transformants, cut with BamHI and Hpal, and run on gel to confirm transformation. This insert was then gel purified and transformed into a convenient expression vector, such as 7921 vector DNA containing a Ubi promoter and pinll terminator.

A preferred DNA sequence is provided in Molecular and General Genetics 216:388-394 (1989). It contains an open reading frame encoding a protein of 239 amino acid residues, with a deduced molecular weight of about 26.3kDa (Calculated as the number of amino acid residues X 110).

Polynucleotides for use in the present invention may be in the form of RNA, such as mRNA, or in the form of DNA, including, for instance, cDNA and genomic DNA obtained by cloning or produced by chemical synthetic techniques or by a combination thereof. The DNA may be double-stranded or single-stranded. Single-stranded DNA may be the coding strand, also known as the sense strand, or it may be the non-coding strand, also referred to as the antisense strand.

The coding sequence which encodes the polypeptide may be identical to the coding sequence of the polynucleotides shown below. It also may be a polynucleotide with a different sequence, which, as a result of the redundancy (degeneracy) of the genetic code, encodes the polypeptide shown below. As discussed more fully below, these alternative coding sequences are an important source of sequences for codon optimization.

Polynucleotides for use in the present invention which encode the polypeptides listed below may include, but are not limited to, the coding sequence for the mature polypeptide, by itself; the coding sequence for the mature polypeptide and additional coding sequences, such as those encoding a leader or secretory sequence, such as a pre-, or pro- or prepro- protein sequence; the coding sequence of the mature polypeptide, with or without the aforementioned additional coding sequences, together with additional, non-coding sequences, including for example, but not limited to, non-coding 5' and 3' sequences, such as the transcribed, non-translated sequences that play a role in transcription (including termination signals, for example), ribosome binding, mRNA stability elements, and additional coding sequences which encode additional amino adds, such as those which provide additional functionalities.

The DNA may also comprise promoter regions which function to direct the transcription of the mRNA encoding heterologous cytokinin metabolic enzymes of this invention. Heterologous is defined as a sequence that is not naturally occurring with the promoter sequence. While the nucleotide sequence is heterologous to the promoter sequence, it may be homologous (native) or heterologous (foreign) to the plant host.

Furthermore, the polypeptide may be fused to a marker sequence, such as a peptide, which facilitates purification of the fused polypeptide. In certain embodiments of this aspect of the invention, the marker sequence is a hexa-histidine peptide, such as the tag provided in the pQE vector (Qiagen, Inc.) and the pET series of vectors (Novagen), among others, many of which are commercially available. As described in Gentz et al., Proc. Nat'l. Acad. Sci., (USA) 86:821-824 (1989), for instance, hexa-histidine provides for convenient purification of the fusion protein. The HA tag may also be used to create fusion proteins and corresponds to an epitope derived of influenza hemagglutinin protein, which has been described by Wilson et al., Cell 37:767 (1984), for instance.

In accordance with the foregoing, the term "polynucleotide encoding a polypeptide" as used herein encompasses polynucleotides which include a sequence encoding a polypeptide for use in the present invention, particularly cytokinin biosynthetic enzymes having the amino acid sequences set out below. The term encompasses polynucleotides that include a single continuous region or discontinuous regions encoding the polypeptide (for example, interrupted by integrated phage or insertion sequence or editing) together with additional regions, that also may contain coding and/or non-coding sequences.

The present invention may use variants of the present polynucleotides which encode for fragments, analogs and derivatives of the polypeptides having the deduced amino acid sequence below. A variant of the polynucleotide may be a naturally occurring variant such as a naturally occurring allelic variant, or it may be a variant that is not known to occur naturally. Such non-naturally occurring variants of the polynucleotide may be made by mutagenesis techniques, including those applied to polynucleotides, cells or organisms.

Among variants in this regard are variants that differ from the aforementioned polynucleotides by nucleotide substitutions, deletions or additions. The substitutions may involve one or more nucleotides. The variants may be altered in coding or non-coding regions or both. Alterations in the coding regions may produce conservative or non-conservative amino add substitutions, deletions or additions.

Polynucleotides encoding polypeptides having the amino acid sequences set out below and variants, analogs, derivatives and fragments thereof may be used in the practice of the invention.

Further particularly preferred in this regard are polynucleotides encoding cytokinin biosynthetic enzyme variants, analogs, derivatives and fragments, and variants, analogs and derivatives of the fragments, which have the amino acid sequences below in which several, a few, 1 to 10, 1 to 5, 1 to 3, 2, 1 or no amino acid residues are substituted, deleted or added, in any combination. Especially preferred among these are silent substitutions, additions and deletions, which do not alter the properties and activities of the cytokinin biosynthetic enzymes. Also especially preferred in this regard are conservative substitutions. Most highly preferred are polynucleotides encoding polypeptides having the amino acid sequence below, without substitutions.

Further preferred embodiments of the invention may use polynucleotides that are greater than 79%, preferably at least 80%, more preferably at least 85% identical to a polynucleotide encoding the ipt polypeptide having the amino acid sequence set out below, and polynucleotides which are complementary to such polynucleotides. Among these particularly preferred polynucleotides, those with at least 90%, 95%, 98% or at least 99% are especially preferred.

Particularly preferred embodiments in this respect, moreover, use polynucleotides which encode polypeptides which retain substantially the same, or even exhibit a increase in, biological function or activity as the mature polypeptide encoded by the polynucleotides set out below.

The present invention may also use polynucleotides that hybridize to the herein above-described sequences. In this regard, the present invention especially relates to polynucleotides which hybridize under stringent conditions to the herein above-described polynucleotides. As herein used, the term "stringent conditions" means hybridization will occur only if there is at least 95% and preferably at least 97% identity between the sequences.

The terms "stringent conditions" or "stringent hybridization conditions" include reference to conditions under which a probe will hybridize to its target sequence, to a detectably greater degree than to other sequences (e.g., at least 2-fold over background). Stringent conditions are sequence-dependent and will be different in different circumstances. By controlling the stringency of the hybridization and/or washing conditions, target sequences can be identified which are 100% complementary to the probe (homologous probing). Alternatively; stringency conditions can be adjusted to allow some mismatching in sequences so that lower degrees of similarity are detected (heterologous probing). Generally, a probe is less than about 1000 nucleotides in length, preferably less than 500 nucleotides in length.

Typically, stringent conditions will be those in which the salt concentration is less than about 1.5 M Na ion, typically about 0.01 to 1.0 M Na ion concentration (or other salts) at pH 7.0 to 8.3 and the temperature is at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulfate) at 37°C. and a wash in 1X to 2X SSC (20X SSC = 3.0 M NaCl/0.3 M trisodium citrate) at 50 to 55°C. Exemplary moderate stringency conditions include hybridization in 40 to 45% formamide. 1 M NaCl. 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0.1X SSC at 60 to 65°C.

Specificity is typically the function of post-hybridization washes, the critical factors being the ionic strength and temperature of the final wash solution. For DNA-DNA hybrids, the Tₘ can be approximated from the equation of Meinkoth and Wahl, Anal. Biochem., 138:267-284 (1984): Tₘ = 81.5 °C + 16.6 (log M) + 0.41 (%GC) - 0.61 (% form) - 500/L: where M is the molarity of monovalent cations, %GC is the percentage of guanosine and cytosine nucleotides in the DNA, % form is the percentage of formamide in the hybridization solution, and L is the length of the hybrid in base pairs. The Tₘ is the temperature (under defined ionic strength and pH) at which 50% of a complementary target sequence hybridizes to a perfectly matched probe. Tₘ is reduced by about 1 °C for each 1% of mismatching; thus, Tₘ, hybridization and/or wash conditions can be adjusted to hybridize to sequences of the desired identity. For example, if sequences with ≥90% identity are sought, the Tₘ can be decreased 10°C. Generally, stringent conditions are selected to be about 5 °C lower than the thermal melting point (Tₘ) for the specific sequence and its complement at a defined ionic strength and pH. However, severely stringent conditions can utilize a hybridization and/or wash at 1, 2, 3, or 4 °C lower than the thermal melting point (Tₘ); moderately stringent conditions can utilize a hybridization and/or wash at 6, 7, 8, 9, or 10°C lower than the thermal melting point (Tₘ); low stringency conditions can utilize a hybridization and/or wash at 11, 12, 13, 14, 15, or 20 °C lower than the thermal melting point (Tₘ). Using the equation, hybridization and wash compositions, and desired Tₘ, those of ordinary skill will understand that variations in the stringency of hybridization and/or wash solutions are inherently described. If the desired degree of mismatching results in a Tₘ of less than 45°C (aqueous solution) or 32 °C (formamide solution) it is preferred to increase the SSC concentration so that a nigher temperature can be used. Hybridization and/or wash conditions can be applied for at least 10, 30, 60, 90, 120, or 240 minutes. An extensive guide to the hybridization of nucleic acids is found in Tijssen, Laboratory Techniques in Biochemistry and Molecular Biology-Hybridization with Nucleic Acid Probes, Part I, Chapter 2 "Overview of principles of hybridization and the strategy of nucleic acid probe assays", Elsevier, New York (1993); and Current Protocols in Molecular Biology, Chapter 2, Ausubel, et al., Eds., Greene Publishing and Wiley-Interscience, New York (1995).

The polynucleotides may encode a polypeptide which is the mature protein plus additional amino or carboxyl-terminal amino acids, or amino acids interior to the mature polypeptide (when the mature form has more than one polypeptide chain, for instance). Such sequences may play a role in processing of a protein from precursor to a mature form, may allow protein transport, may lengthen or shorten protein half-life or may facilitate manipulation of a protein for assay or production, among other things. As generally is the case *in vivo,* the additional amino acids may be processed away from the mature protein by cellular enzymes.

A precursor protein, having the mature form of the polypeptide fused to one or more prosequences, may be an inactive form of the polypeptide. When prosequences are removed, such inactive precursors generally are activated. Some or all of the prosequences may be removed before activation. Generally, such precursors are called proproteins.

In sum, a polynucleotide used in the present invention may encode a mature protein, a mature protein plus a leader sequence (which may be referred to as a preprotein), a precursor of a mature protein having one or more prosequences which are not the leader sequences of a preprotein, or a preproprotein, which is a precursor to a proprotein, having a leader sequence and one or more prosequences, which generally are removed during processing steps that produce active and mature forms of the polypeptide.

### Polypeptides

The present invention may use relates to polypeptides that have the deduced amino acid sequences below.

The invention also relates to fragments, analogs and derivatives of these polypeptides. The terms "fragment," "derivative" and "analog", when referring to the polypeptides, mean a polypeptide which retains essentially the same biological function or activity as such polypeptide. Fragments, derivatives and analogs that retain at least 90% of the activity of the native cytokinin biosynthetic enzymes are preferred. Fragments, derivatives and analogs that retain at least 95% of the activity of the native polypeptides are preferred. Thus, an analog includes a proprotein which can be activated by cleavage of the proprotein portion to produce an active mature polypeptide.

The polypeptide used in the practice of the present invention may be a recombinant polypeptide, a natural polypeptide or a synthetic polypeptide. In certain preferred embodiments it is a recombinant polypeptide.

The fragment, derivative or analog of the polypeptides below may be (i) one in which one or more of the amino acid residues are substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which the mature polypeptide is fused with another compound, such as a compound to increase the half-life of the polypeptide (for example, polyethylene glycol), or (iv) one in which the additional amino acids are fused to the mature polypeptide, such as a leader or secretory sequence or a sequence which is employed for purification of the mature polypeptide or a proprotein sequence. Such fragments, derivatives and analogs are deemed to be obtained by those of ordinary skill in the art, from the teachings herein.

The particularly preferred embodiments of the invention may use polypeptides having the amino acid sequence of cytokinin biosynthetic enzymes set out below, variants, analogs, derivatives and fragments thereof, and variants, analogs and derivatives of the fragments.

Among preferred variants are those that vary from a reference by conservative amino acid substitutions. Such substitutions are those that substitute a given amino acid in a polypeptide by another amino acid of like characteristics. Typically seen as conservative substitutions are the replacements, one for another, among the aliphatic amino acids Ala, Val, Leu and Ile; interchange of the hydroxyl residues Ser and Thr, exchange of the acidic residues Asp and Glu, substitution between the amide residues Asn and Gln, exchange of the basic residues Lys and Arg and replacements among the aromatic residues Phe, Tyr.

Further particularly preferred in this regard are variants, analogs, derivatives and fragments, and variants, analogs and derivatives of the fragments, having the amino acid sequence below, in which several, a few, 1 to 10, 1 to 5, 1 to 3, 2, 1 or no amino acid residues are substituted, deleted or added, in any combination. Especially preferred among these are silent substitutions, additions and deletions, which do not alter the properties and activities of the cytokinin biosynthetic enzymes. Also especially preferred in this regard are conservative substitutions. Most highly preferred are polypeptides having the amino acid sequences below without substitutions.

The polypeptides and polynucleotides are preferably provided in an isolated form, and may be purified to homogeneity.

### Vectors, Host Cells. Expression

The present invention may use vectors comprising the polynucleotides of the present invention, host cells that incorporate said vectors.

### Vectors

The vector may be, for example, a plasmid vector, a single or double-stranded phage vector, a single or double-stranded RNA or DNA viral vector. Such vectors may be introduced into cells as polynucleotides, preferably DNA, by well known techniques for introducing DNA and RNA into cells. The vectors, in the case of phage and viral vectors also may be and preferably are introduced into cells as packaged or encapsidated virus by well known techniques for infection and transduction. Viral vectors may be replication competent or replication defective. In the latter case, viral propagation generally will occur only in complementing host cells.

Preferred among vectors, in certain respects, are those for expression of polynucleotides and polypeptides of use in the practice of the present invention. Generally, such vectors comprise cis-acting control regions effective for expression in a host operatively linked to the polynucleotide to be expressed. Appropriate trans-acting factors are supplied by the host, supplied by a complementing vector or supplied by the vector itself upon introduction into the host.

In certain preferred embodiments in this regard, the vectors provide for preferred expression. Such preferred expression may be inducible expression or temporally limited or restricted to predominantly certain types of cells or any combination of the above. Particularly preferred among inducible vectors are vectors that can be induced for expression by environmental factors that are easy to manipulate, such as temperature and nutrient additives. A variety of vectors suitable to this aspect of the invention, including constitutive and inducible expression vectors for use in prokaryotic and eukaryotic hosts, are well known and employed routinely by those of skill in the art. Such vectors include, among others, chromosomal, episomal and virus-derived vectors, *e.g*., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids and binaries used for Agrobacterium-mediated transformations. All may be used for expression in accordance with this aspect of the present invention.

The following vectors, which are commercially available, are provided by way of example. Among vectors preferred for use in bacteria are pQE70, pQE60 and pQE-9, available from Qiagen; pBS vectors, Phagescript vectors, Bluescript vectors, pNH8A, pNH16a, pNH18A, pNH46A, available from Stratagene; and ptrc99a, pKK223-3, pKK233-3, pDR540, pRIT5 available from Pharmacia. Among preferred eukaryotic vectors are pWLNEO, pSV2CAT, pOG44, pXT1 and pSG available from Stratagene; and pSVK3, pBPV, pMSG and pSVL available from Pharmacia. Useful plant binaries vectors include BIN19 and its derivatives available from Clontech. These vectors are listed solely by way of illustration of the many commercially available and well known vectors that are available to those of skill in the art for use in accordance with this aspect of the present invention. It will be appreciated that any other plasmid or vector suitable for, for example, introduction, maintenance, propagation or expression of a polynucleotide or polypeptide of the invention in a host may be used in this aspect of the invention, several of which are disclosed in more detail below.

In general, expression constructs will contain sites for transcription initiation and termination, and, in the transcribed region, a ribosome binding site for translation. The coding portion of the mature transcripts expressed by the constructs will include a translation initiating AUG at the beginning and a termination codon appropriately positioned at the end of the polypeptide to be translated.

In addition, the constructs may contain control regions that regulate as well as engender expression. Generally, in accordance with many commonly practiced procedures, such regions will operate by controlling transcription, such as transcription factors, repressor binding sites and termination, among others. For secretion of the translated protein into the lumen of the endoplasmic reticulum, into the periplasmic space or into the extracellular environment, appropriate secretion signals may be incorporated into the expressed polypeptide. These signals may be endogenous to the polypeptide or they may be heterologous signals.

Transcription of the DNA encoding the polypeptides of use in the practice of the present invention by higher eukaryotes may be increased by inserting an enhancer sequence into the vector. Enhancers are cis-acting elements of DNA, usually about from 10 to 300 bp that act to increase transcriptional activity of a promoter in a given host cell-type. Examples of enhancers include the SV40 enhancer, which is located on the late side of the replication origin at bp 100 to 270, the cytomegalovirus early promoter enhancer, the polyoma enhancer on the late side of the replication origin, and adenovirus enhancers. Additional enhancers useful in the invention to increase transcription of the introduced DNA segment, include, *inter alia,* viral enhancers like those within the 35S promoter, as shown by Odell et al., Plant Mol. Biol. 10:263-72 (1988), and an enhancer from an opine gene as described by Fromm et al., Plant Cell 1:977 (1989).

Termination regions also facilitate effective expression by ending transcription at appropriate points. Useful terminators for practicing this invention include, but are not limited to, pinII (*See* An et al., Plant Cell 1(1):115-122 (1989)), glb1 (*See* Genbank Accession #L22345), gz (See gzw64a terminator, Genbank Accession #S78780), and nos. Temporally acting promoters are used by this invention. Promoters that act from 0-25 days after pollination (DAP) are preferred, as are those acting from 4-21, 4-12, or 8-12 DAP. Specifically the ltp2 promoter is used.

Vectors for propagation and expression generally will include selectable markers. Such markers also may be suitable for amplification or the vectors may contain additional markers for this purpose. In this regard, the expression vectors preferably contain one or more selectable marker genes to provide a phenotypic trait for selection of transformed host cells. Preferred markers include dihydrofolate reductase or neomycin resistance for eukaryotic cell culture, and tetracycline or ampicillin resistance genes for culturing *E. coli* and other prokaryotes. Kanamycin and herbicide resistance genes (PAT and BAR) are generally useful in plant systems.

Selectable marker genes, in physical proximity to the introduced DNA segment, are used to allow transformed cells to be recovered by either positive genetic selection or screening. The selectable marker genes also allow for maintaining selection pressure on a transgenic plant population, to ensure that the introduced DNA segment, and its controlling promoters and enhancers, are retained by the transgenic plant.

Many of the commonly used positive selectable marker genes for plant transformation have been isolated from bacteria and code for enzymes that metabolically detoxify a selective chemical agent which may be an antibiotic or a herbicide. Other positive selection marker genes encode an altered target which is insensitive to the inhibitor.

A preferred selection marker gene for plant transformation is the BAR or PAT gene, which is used with the selecting agent bialaphos. Spencer et al., J. Thero. Appl'd Genetics 79:625-631 (1990). Another useful selection marker gene is the neomycin phosphotransferase II *(nptII)* gene, isolated from Tn5, which confers resistance to kanamycin when placed under the control of plant regulatory signals. Fraley et al., Proc. Nat'l Acad. Sci. (USA) 80:4803 (1983). The hygromycin phosphotransferase gene, which confers resistance to the antibiotic hygromycin, is a further example of a useful selectable marker. Vanden Elzen et al., Plant Mol. Biol. 5:299 (1985). Additional positive selectable markers genes of bacterial origin that confer resistance to antibiotics include gentamicin acetyl transferase, streptomycin phosphotransferase, aminoglycoside-3'-adenyl transferase and the bleomycin resistance determinant. Hayford et al., Plant Physiol. 86:1216 (1988); Jones et al., Mol. Gen. Genet. 210:86 (1987); Svab et al., Plant Mol. Biol. 14:197 (1990); Hille et al., Plant Mol. Biol. 7:171 (1986).

Other positive selectable marker genes for plant transformation are not of bacterial origin. These genes include mouse dihydrofolate reductase, plant 5-enolpyruvylshikimate-3-phosphate synthase and plant acetolactate synthase. Eichholtz et al., Somatic Cell Mol. Genet. 13:67 (1987); Shah et al., Science 233:478 (1986); Charest et al., Plant Cell Rep. 8:643 (1990).

Another class of useful marker genes for plant transformation with the DNA sequence requires screening of presumptively transformed plant cells rather than direct genetic selection of transformed cells for resistance to a toxic substance such as an antibiotic. These genes are particularly useful to quantitate or visualize the spatial pattern of expression of the DNA sequence in specific tissues and are frequently referred to as reporter genes because they can be fused to a gene or gene regulatory sequence for the investigation of gene expression. Commonly used genes for screening presumptively transformed cells include β-glucuronidase (GUS), β-galactosidase, luciferase, and chloramphenicol acetyltransferase. Jefferson, Plant Mol. Biol. Rep. 5:387 (1987); Teeri et al., EMBO J. 8:343 (1989); Koncz et al., Proc. Nat'l Acad. Sci. (USA) 84:131 (1987); De Block et al., EMBO J. 3:1681 (1984). Another approach to the identification of relatively rare transformation events has been use of a gene that encodes a dominant constitutive regulator of the *Zea mays* anthocyanin pigmentation pathway(Ludwig et al., Science 247:449 (1990)).

The appropriate DNA sequence may be inserted into the vector by any of a variety of well-known and routine techniques. In general, a DNA sequence for expression is joined to an expression vector by cleaving the DNA sequence and the expression vector with one or more restriction endonucleases and then joining the restriction fragments together using T4 DNA ligase. The sequence may be inserted in a forward or reverse orientation. Procedures for restriction and ligation that can be used to this end are well known and routine to those of skill. Suitable procedures in this regard, and for constructing expression vectors using alternative techniques, which also are well known and routine to those of skill, are set forth in great detail in Sambrook et al., MOLECULAR CLONING, A LABORATORY MANUAL, 2nd Ed.; Cold Spring Harbor Laboratory Press, Cold Spring Harbor, New York (1989).

A polynucleotide of the invention, encoding the heterologous structural sequence of a polypeptide of the invention, generally will be inserted into the vector using standard techniques so that it is operably linked to the promoter for expression. The polynucleotide will be positioned so that the transcription start site is located appropriately 5' to a ribosome binding site. The ribosome binding site will be 5' to the AUG that initiates translation of the polypeptide to be expressed. Generally, there will be no other open reading frames that begin with an initiation codon, usually AUG, and lie between the ribosome binding site and the initiation codon. Also, generally, there will be a translation stop codon at the end of the polypeptide and there will be a polyadenylation signal in constructs for use in eukaryotic hosts. Transcription termination signal appropriately disposed at the 3' end of the transcribed region may also be included in the polynucleotide construct.

The vector containing the appropriate DNA sequence as described elsewhere herein, as well as an appropriate promoter, and other appropriate control sequences, may be introduced into an appropriate host using a variety of well known techniques suitable to expression therein of a desired polypeptide. Also described are host cells containing the above-described constructs discussed. The host cell can be a higher eukaryotic cell, such as a mammalian or plant cell, or a lower eukaryotic cell, such as a yeast cell, or the host cell can be a prokaryotic cell, such as a bactenal cell.

Introduction of the construct into the host cell can be effected by calcium phosphate transfection, DEAE-dextran mediated transfection, microinjection, cationic lipid-mediated transfection, electroporation, transduction, scrape loading, ballistic introduction, infection or other methods. Such methods are described in many standard laboratory manuals, such as Davis et al., BASIC METHODS IN MOLECULAR BIOLOGY, (1986) and Sambrook et al., MOLECULAR CLONING: A LABORATORY MANUAL, 2nd Ed., Cold Spring Harbor Laboratory Press, Cold Spring Harbor, N.Y. (1989).

Representative examples of appropriate hosts include bacterial cells, such as streptococci, staphylococci, *E*. *coli,* streptomyces and *Salmonella typhirmurium* cells; fungal cells, such as yeast cells and *Aspergillus* cells; insect cells such as *Drosophila* S2 and *Spodoptera* Sf9 cells; animal cells such as CHO, COS and Bowes melanoma cells; and plant cells. The plants cells may be derived from a broad range of plant types, particularly monocots such as the species of the Family *Graminiae* including *Sorghum bicolor* and *Zea mays.* The isolated nucleic acid and proteins of the present invention can also be used in species from the genera: *Cucurbita, Rosa, Vitis, Juglans, Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersicon, Nicotiana, Solanum, Petunia, Digitalis, Majorana, Cichorium, Helianthus, Lactuca, Bromus, Asparagus, Antirrhinum, Heterocallis, Nemesis, Pelargonium, Panieum, Pennisetum, Ranunculus, Senecio, Salpiglossis, Cucumis, Browaalia, Glycine, Pisum, Phaseolus, Lolium, Oryza, Avena, Hordeum, Secale,* and *Triticum.*

### Plant Transformation Methods:

Isolated nucleic acids used in the practice of the present invention can be introduced into plants according to techniques known in the art. Generally, recombinant expression cassettes as described above and suitable for transformation of plant cells are prepared. Techniques for transforming a wide variety of higher plant species are well known and described in the technical, scientific, and patent literature. See, for example, Weising et al., Ann. Rev. Genet. 22: 421-477 (198a). For example, the DNA construct may be introduced directly into the genomic DNA of the plant cell using techniques such as electroporation, PEG poration, particle bombardment, silicon fiber delivery, or microinjection of plant cell protoplasts or embryogenic callus. Alternatively, the DNA constructs may be combined with suitable T-DNA flanking regions and introduced into a conventional *Agrobacterium tumefaciens* host vector. The virulence functions of the *Agrobacterium tumefaciens* host will direct the insertion of the construct and adjacent marker into the plant cell DNA when the cell is infected by the bacteria. See, U.S. Patent No. 5,591,616.

The introduction of DNA constructs using polyethylene glycol precipitation is described in Paszkowski et al., Embo J. 3: 2717-2722 (1984). Electroporation techniques are described in Fromm et al., Proc. Natl. Acad. Sci (USA) 82: 5824 (1985). Ballistic transformation techniques are described in Klein et al., Nature 327: 70-73 (1987) and by Tomes, D. et al., IN: Plant Cell, Tissue and Organ Culture: Fundamental Methods, Eds. O.L. Gamborg and G.C. Phillips, Chapter 8, pgs. 197-213 (1995). (See also Tomes et al., U.S. Patent 5,886,244.)
*Agrobacterium* tumefaciens-meditated transformation techniques are well described in the scientific literature. See, for example Horsch et al., Science 233: 496-498 (1984), and Fraley et al., Proc. Natl. Acad. Sci (USA) 80: 4803 (1983). Although *Agrobacterium* is useful primarily in dicots, certain monocots can be transformed by *Agrobacterium.* For instance, *Agrobacterium* transformation of maize is described in U.S. Patent No. 5,550,318.

Other methods of transfection or transformation include (1) *Agrobacterium* rhizogenes-mediated transformation (see, e.g., Lichtenstein and Fuller In: Genetic Engineering, vol. 6, PWJ Rigby, Ed., London, Academic Press, 1987; and Lichtenstein, C. P., and Draper, J,. In: DNA Cloning, Vol. II, D. M. Glover, Ed., Oxford, IRI Press, 1985),Application PCT/US87/02512 (WO 88/02405 published Apr. 7, 1988) describes the use of *A.rhizogenes* strain A4 and its Ri plasmid along with *A*. *tumefaciens* vectors pARC8 or pARC16 (2) liposome-mediated DNA uptake (see, e.g., Freeman et al., Plant Cell Physiol. 25: 1353, 1984), (3) the vortexing method (see, e.g., Kindle, Proc. Nat'l. Acad. Sci.(USA) 87: 1228, (1990).

DNA can also be introduced into plants by direct DNA transfer into pollen as described by Zhou et al., Methods in Enzymology, 101:433 (1983); D. Hess, Intern. Rev. Cytol., 107:367 (1987); Luo et al., Plant Mol. Biol. Reporter, 6:165(1988). Expression of polypeptide coding genes can be obtained by injection of the DNA into reproductive organs of a plant as described by Pena et al., Nature 325:274 (1987). DNA can also be injected directly into the cells of immature embryos and the rehydration of desiccated embryos as described by Neuhaus et al., Theor. Appl. Genet., 75:30 (1987); and Benbrook et al., in Proceedings Bio Expo. 1986, Butterworth, Stoneham, Mass., pp. 27-54 (1986). A variety of plant viruses that can be employed as vectors are known in the art and include cauliflower mosaic virus (CaMV), geminivirus, brome mosaic virus, and tobacco mosaic virus.

### Regeneration of Transformed Plants

Transformed plant cells which are derived by any of the above transformation techniques can be cultured to regenerate a whole plant which possesses the transformed genotype. Such regeneration techniques often rely on manipulation of certain phytohormones in a tissue culture growth medium, typically relying on a biocide and/or herbicide marker which has been introduced together with a polynucleotide used in the practice of the present invention. For transformation and regeneration of maize see, Gordon-Kamm et al., The Plant Cell, 2:603-618 (1990).

Plants cells transformed with a plant expression vector can be regenerated, e.g., from single cells, callus tissue or leaf discs according to standard plant tissue culture techniques. It is well known in the art that various cells, tissues, and organs from almost any plant can be successfully cultured to regenerate an entire plant. Plant regeneration from cultured protoplasts is described in Evans et al., Protoplasts Isolation and Culture, Handbook of Plant Cell Culture, Macmillilan Publishing Company, New York, pp. 124-176 (1983); and Binding, Regeneration of Plants, Plant Protoplasts, CRC Press, Boca Raton, pp. 21-73 (1985).

The regeneration of plants containing the foreign gene introduced by *Agrobacterium* from leaf explants can be achieved as described by Horsch et al., Science, 227:1229-1231 (1985). In this procedure, transformants are grown in the presence of a selection agent and in a medium that induces the regeneration of shoots in the plant species being transformed as described by Fraley et al., Proc. Nat'l. Acad. Sci. (U.S.A)., 80:4803 (1983). This procedure typically produces shoots within two to four weeks and these transformant shoots are then transferred to an appropriate root-inducing medium containing the selective agent and an antibiotic to prevent bacterial growth. Transgenic plants of the present invention may be fertile or sterile.

Regeneration can also be obtained from plant callus, explants, organs, or parts thereof. Such regeneration techniques are described generally in Klee et al., Ann. Rev. of Plant Phys. 38: 467-486 (1987). The regeneration of plants from either single plant protoplasts or various explants is well known in the art. See, for example, Methods for Plant Molecular Biology, A. Weissbach and H. Weissbach, eds., Academic Press, Inc., San Diego, Calif. (1988). This regeneration and growth process includes the steps of selection of transformant cells and shoots, rooting the transformant shoots and growth of the plantlets in soil. For maize cell culture and regeneration see generally, The Maize Handbook, Freeling and Walbot, Eds., Springer, New York (1994); Corn and Corn Improvement, 3rd edition, Sprague and Dudley Eds., American Society of Agronomy, Madison, Wisconsin (1988).

One of skill will recognize that after the recombinant expression cassette is stably incorporated in transgenic plants and confirmed to be operable, it can be introduced into other plants by sexual crossing. Any of a number of standard breeding techniques can be used, depending upon the species to be crossed.

In vegetatively propagated crops, mature transgenic plants can be propagated by the taking of cuttings or by tissue culture techniques to produce multiple identical plants. Selection of desirable transgenics is made and new varieties are obtained and propagated vegetatively for commercial use. In seed-propagated crops, mature transgenic plants can be self-crossed to produce a homozygous inbred plant. The inbred plant produces seed containing the newly introduced heterologous nucleic acid. These seeds can be grown to produce plants that would produce the selected phenotype.

Parts obtained from the regenerated plant, such as flowers, seeds, leaves, branches, fruit, and the like are included in the invention, provided that these parts comprise cells comprising the isolated nucleic acid of the present invention. Progeny and variants, and mutants of the regenerated plants are also included within the scope of the invention, provided that these parts comprise the introduced nucleic acid sequences.

Transgenic plants expressing the selectable marker can be screened for transmission of the nucleic acid of the present invention by, for example, standard immunoblot and DNA detection techniques. Transgenic lines are also typically evaluated on levels of expression of the heterologous nucleic acid. Expression at the RNA level can be determined initially to identify and quantitate expression-positive plants. Standard techniques for RNA analysis can be employed and include PCR amplification assays using oligonucleotide primers designed to amplify only the heterologous RNA templates and solution hybridization assays using heterologous nucleic acid-specific probes. The RNA-positive plants can then be analyzed for protein expression by Western immunoblot analysis using the specifically reactive antibodies of the present invention. In addition, *in situ* hybridization and immunocytochemistry according to standard protocols can be done using heterologous nucleic acid specific polynucleotide probes and antibodies, respectively, to localize sites of expression within transgenic tissue. Generally, a number of transgenic lines are usually screened for the incorporated nucleic acid to identify and select plants with the most appropriate expression profiles.

A preferred embodiment is a transgenic plant that is homozygous for the added heterologous nucleic acid; i.e., a transgenic plant that contains two added nucleic acid sequences, one gene at the same locus on each chromosome of a chromosome pair. A homozygous transgenic plant can be obtained by sexually mating (selfing) a heterozygous transgenic plant that contains a single added heterologous nucleic acid, germinating some of the seed produced and analyzing the resulting plants produced for altered expression of a polynucleotide of the present invention relative to a control plant (i.e., native, non-transgenic). Back-crossing to a parental plant and out-crossing with a non- transgenic plant are also contemplated.

It is also expected that the transformed plants will be used in traditional breeding programs, including TOPCROSS pollination systems as disclosed in US 5,706,603 and US 5,704,160.

### Examples

The present invention is further described by the following examples.

Certain terms used herein are explained in the foregoing glossary.

All examples were carried out using standard techniques, which are well known and routine to those of skill in the art, except where otherwise described in detail. Routine molecular biology techniques of the following examples can be carried out as described in standard laboratory manuals, such as Sambrook et al., MOLECULAR CLONING: A LABORATORY MANUAL, 2nd Ed.: Cold Spring Harbor Laboratory Press, Cold Spring Harbor, N.Y. (1989).

All parts or amounts set out in the following examples are by weight, unless otherwise specified.

Unless otherwise stated, size separation of fragments in the examples below was carried out using standard techniques of agarose and polyacrylamide gel electrophoresis ("PAGE") in Sambrook et al., MOLECULAR CLONING: A LABORATORY MANUAL, 2nd Ed., Cold Spring Harbor Laboratory Press, Cold Spring Harbor, N.Y. (1989) and numerous other references such as, for instance, by Goeddel et al., Nucleic Acids Res. 8:4057 (1980).

Unless described otherwise, ligations were accomplished using standard buffers, incubation temperatures and times, approximately equimolar amounts of the DNA fragments to be ligated and approximately 10 units of T4 DNA ligase ("ligase") per 0.5 microgram of DNA.

### Example 1: Construction of vectors system for temporal and spatial seed preferred expression of cytokinin biosynthetic enzymes

### Construction of PHP11404 and PHP11550.

PHP 11404 was used with the biolistics-mediated transformation protocol, The plasmid has all the features of the Agro version. The plasmid that was actually used with the Agro-mediated transformation protocols was PHP11550.

Using the plasmid PHP9063 (pUBI:UBIINTRON1:iptpinII 3'), an NcoI restriction site was created at the start codon of ipt using site-directed mutagenesis (specifically, the MORPH™ Kit of 5 Prime → 3 Prime, Inc.). The resulting plasmid was designated PHP11362. The ipt coding sequence was then moved as a 724 bp NcoI/HpaI fragment into PHP8001 (BamHl-cut, treated with Klenow to fill in the overhang to a blunt then cut with NcoI, 4.9 kb) to give PHP11401. PHP8001 contains the GZ-W64A promoter and terminator from the 27 KD zein gene of Z. mays (Genbank Accession # S78780). PHP11401 was digested with PacI + Kpnl and a 1.35 kb fragment inserted into PHP11287 (PacI/KpnI-digested, 10.87 kb) to give PHP11404. PHP11287 is a T-DNA vector that already carries the above-described pUBI:UBIINTRON1:maize-optimized PAT:35S 3' selectable marker. After triparental mating the cointegrate of PHP11404/PHP10523 was designated PHP11550.

### Construction of PHP12425

Plasmid PHP11404 (described above) was used as a starting plasmid to replace the GZ-W64A promoter with the LTP2 promoter from *H. vulgare.* PHP11404 DNA was digested with NotI and KpnI (9.46 kb fragment) and separately with Ncol plus Kpnl (1.24 kb fragment). These two fragments were mixed with a 1.52 kb NotI/NcoI fragment from PHP8219 containing the LTP2 promoter and ligated. The resulting plasmid product was designated PHP12333. Triparental mating of this plasmid into *A*. *tumefaciens* LBA4404 (PHP10523) gave the cointegrate plasmid PHP12425.

### Triparental mating and selectable marker 35s:bar:pinII:

All vectors were constructed using standard molecular biology techniques. The T-DNA region for transformation consists of the T-DNA border sequences flanking a reporter gene and a selectable marker. The reporter is inserted proximal to the right T-DNA border and consists of the 2.0 kb PstI fragment of the maize ubiquitin promoter Ubi-1 (Christensen et al., 1992) with flanking 5' HindIII and 3' BamHI restriction sites. The ubiquitin promoter was ligated to the 5' BamHI site of a beta-glucuronidase (GUS) reporter gene (Jefferson et al., 1986), containing the second intron from potato ST-LS1 (Vancenneyt et al., 1990). The potato proteinase II (pinII) terminator (bases 2 to 310 from An et al., Plant Cell 1(1):115-122 (1989)) was blunt-end ligated downstream of the GUS coding sequence. On the 3' end of the terminator is a NotI restriction site.

The selectable marker consists of an enhanced cauliflower mosaic virus 35S promoter (bases -421 to -90 and -421 to +2 from Gardner, R.C., et al., Nucl. Acids Res. 9:2871-88 (1981).)) with a flanking 5' NotI site and 3' Pstl site. A PstI/SaII fragment containing the 79 bp tobacco mosaic virus leader (Gallie, D.R., et al., Nucl. Acids Res. 15:3257-73 (1987).)) is inserted downstream of the promoter followed by a SaII/BamHI fragment containing the first intron of maize alcohol dehydrogenase ADH1-S (Dennis et al., 1984). The BAR coding sequence (Thompson, C.J., et al., Embo J. 6:2519-23 (1987).)) was cloned into the BamHI site, with the pinII terminator ligated downstream. The pinII signal is flanked by a 3' SacI site.

The T-DNA of PHP8904 was integrated into the super binary plasmid pSB1 (Ishida et al. 1996) by homologous recombination between the two plasmids. E. coli strain HB101 containing PHP8904 was mated with Agrobacterium strain LBA4404 harboring pSB1 to create the cointegrate plasmid in Agrobacterium designated LBA4404(PHP10525) (by the method Ditta, G., et al., Proc. Natl. Acad. Sci. USA 77:7347-51 (1980).) LBA4404(PHP10525) was selected for by Agrobacterium resistance to spectinomycin and verified as a recombinant by a SaII restriction digest of the plasmid.

### Example 2: Transformation of Maize

### Biolistics:

The inventive polynucleotides contained within a vector are transformed into embryogenic maize callus by particle bombardment, generally as described by Tomes, D. et al., IN: Plant Cell, Tissue and Organ Culture: Fundamental Methods, Eds. O.L. Gamborg and G.C. Phillips, Chapter 8, pgs. 197-213 (1995) and is briefly outlined below. Transgenic maize plants are produced by bombardment of embryogenically responsive immature embryos with tungsten particles associated with DNA plasmids. The plasmids consist of a selectable and an unselected structural gene.

### Preparation of Particles:

Fifteen mg of tungsten particles (General Electric), 0.5 to 1.8 µ, preferably 1 to 1.8 µ, and most preferably 1 µ, are added to 2 ml of concentrated nitric acid. This suspension was sonicated at 0°C for 20 minutes (Branson Sonifier Model 450, 40% output, constant duty cycle). Tungsten particles are pelleted by centrifugation at 10000 rpm (Biofuge) for one minute, and the supernatant is removed. Two milliliters of sterile distilled water are added to the pellet, and brief sonication is used to resuspend the particles. The suspension is pelleted, one milliliter of absolute ethanol is added to the pellet, and brief sonication is used to resuspend the particles. Rinsing, pelleting, and resuspending of the particles is performed two more times with sterile distilled water, and finally the particles are resuspended in two milliliters of sterile distilled water. The particles are subdivided into 250-ml aliquots and stored frozen.

### Preparation of Particle-Plasmid DNA Association:

The stock of tungsten particles are sonicated briefly in a water bath sonicator (Branson Sonifier Model 450, 20% output, constant duty cycle) and 50 ml is transferred to a microfuge tube. All the vectors were cis: that is the selectable marker and the gene of interest were on the same plasmid. These vectors were then transformed either singly or in combination.

Plasmid DNA was added to the particles for a final DNA amount of 0.1 to 10 µg in 10 µL total volume, and briefly sonicated. Preferably, 10 µg (1 µg/µL in TE buffer) total DNA is used to mix DNA and particles for bombardment. Specifically, 1.0 µg of PHP 11404, 11466, and/or 11467 (1µg/µL), where any cytokinin biosynthetic enzyme polynucleotide can replace ipt were used per bombardment. Fifty microliters (50 µL) of sterile aqueous 2.5 M CaCl₂ are added, and the mixture is briefly sonicated and vortexed. Twenty microliters (20 µL) of sterile aqueous 0.1 M spermidine are added and the mixture is briefly sonicated and vortexed. The mixture is incubated at room temperature for 20 minutes with intermittent brief sonication. The particle suspension is centrifuged, and the supernatant is removed. Two hundred fifty microliters (250 µL) of absolute ethanol are added to the pellet, followed by brief sonication. The suspension is pelleted, the supernatant is removed, and 60 ml of absolute ethanol are added. The suspension is sonicated briefly before loading the particle-DNA agglomeration onto macrocarriers.

### Preparation of Tissue

Immature embryos of maize variety High Type II are the target for particle bombardment-mediated transformation. This genotype is the F₁ of two purebred genetic lines, parents A and B, derived from the cross of two known maize inbreds, A188 and B73. Both parents are selected for high competence of somatic embryogenesis, according to Armstrong et al., Maize Genetics Coop. News 65:92 (1991).

Ears from F, plants are selfed or sibbed, and embryos are aseptically dissected from developing caryopses when the scutellum first becomes opaque. This stage occurs about 9-13 days post-pollination, and most generally about 10 days post-pollination, depending on growth conditions. The embryos are about 0.75 to 1.5 millimeters long. Ears are surface sterilized with 20-50% Clorox for 30 minutes, followed by three rinses with sterile distilled water.

Immature embryos are cultured with the scutellum oriented upward, on embryogenic induction medium comprised of N6 basal salts, Eriksson vitamins, 0.5 mg/l thiamine HCl, 30 gm/l sucrose, 2.88 gm/l L-proline, 1 mg/l 2,4-dichlorophenoxyacetic acid, 2 gm/l Gelrite, and 8.5 mg/l AgNO₃. Chu et al., Sci. Sin. 18:659 (1975); Eriksson, Physiol. Plant 18:976 (1965). The medium is sterilized by autoclaving at 121°C for 15 minutes and dispensed into 100 X 25 mm Petri dishes. AgNO₃ is filter-sterilized and added to the medium after autoclaving. The tissues are cultured in complete darkness at 28°C. After about 3 to 7 days, most usually about 4 days, the scutellum of the embryo swells to about double its original size and the protuberances at the coleorhizal surface of the scutellum indicate the inception of embryogenic tissue. Up to 100% of the embryos display this response, but most commonly, the embryogenic response frequency is about 80%.

When the embryogenic response is observed, the embryos are transferred to a medium comprised of induction medium modified to contain 120 gm/l sucrose. The embryos are oriented with the coleorhizal pole, the embryogenically responsive tissue, upwards from the culture medium. Ten embryos per Petri dish are located in the center of a Petri dish in an area about 2 cm in diameter. The embryos are maintained on this medium for 3-16 hour, preferably 4 hours, in complete darkness at 28°C just prior to bombardment with particles associated with plasmid DNAs containing the selectable and unselectable marker genes.

To effect particle bombardment of embryos, the particle-DNA agglomerates are accelerated using a DuPont PDS-1000 particle acceleration device. The particle-DNA agglomeration is briefly sonicated and 10 ml are deposited on macrocarriers and the ethanol is allowed to evaporate. The macrocarrier is accelerated onto a stainless-steel stopping screen by the rupture of a polymer diaphragm (rupture disk). Rupture is effected by pressurized helium. The velocity of particle-DNA acceleration is determined based on the rupture disk breaking pressure. Rupture disk pressures of 200 to 1800 psi are used (equivalent to 1.38 mPa to 12.41 mPa in 5.1.units), with 650 to 1100 psi (equivalent to 4.48 to 7.58 mPa in 5.1.units) being preferred, and about 900 psi (equivalent to 7.58 mPa in 5.1.units) being most highly preferred. Multiple disks are used to effect a range of rupture pressures.

The shelf containing the plate with embryos is placed 5.1 cm below the bottom of the macrocarrier platform (shelf #3). To effect particle bombardment of cultured immature embryos, a rupture disk and a macrocarrier with dried particle-DNA agglomerates are installed in the device. The He pressure delivered to the device is adjusted to 200 psi (equivalent to 1.38 mPa) above the rupture disk breaking pressure. A Petri dish with the target embryos is placed into the vacuum chamber and located in the projected path of accelerated particles. A vacuum is created in the chamber, preferably about 28 in Hg (equivalent to 3.05 mPa in 5.1.units). After operation of the device, the vacuum is released and the Petri dish is removed.

Bombarded embryos remain on the osmotically-adjusted medium during bombardment, and 1 to 4 days subsequently. The embryos are transferred to selection medium comprised of N6 basal salts, Eriksson vitamins, 0.5 mg/1 thiamine HCl, 30 gm/l sucrose, 1 mg/l 2,4-dichlorophenoxyacetic acid, 2 gm/l Gelrite, 0.85 mg/l Ag NO₃ and 3 mg/l bialaphos (Herbiace, Meiji). Bialaphos is added filter-sterilized. The embryos are subcultured to fresh selection medium at 10 to 14 day intervals. After about 7 weeks, embryogenic tissue, putatively transformed for both selectable and unselected marker genes, proliferates from about 7% of the bombarded embryos. Putative transgenic tissue is rescued, and that tissue derived from individual embryos is considered to be an event and is propagated independently on selection medium. Two cycles of clonal propagation are achieved by visual selection for the smallest contiguous fragments of organized embryogenic tissue.

A sample of tissue from each event is processed to recover DNA. The DNA is restricted with a restriction endonuclease and probed with primer sequences designed to amplify DNA sequences overlapping the cytokinin biosynthetic enzymes and non- cytokinin biosynthetic enzyme portion of the plasmid. Embryogenic tissue with amplifiable sequence is advanced to plant regeneration.

For regeneration of transgenic plants, embryogenic tissue is subcultured to a medium comprising MS salts and vitamins (Murashige & Skoog, Physiol. Plant 15: 473 (1962)). 100 mg/l myo-inositol, 60 gm/l sucrose, 3 gm/l Gelrite, 0.5 mg/l zeatin, 1 mg/l indole-3-acetic acid, 26.4 ng/l cis-trans-abscisic acid, and 3 mg/l bialaphos in 100 X 25 mm Petri dishes, and is incubated in darkness at 28°C until the development of well-formed, matured somatic embryos can be seen. This requires about 14 days. Well-formed somatic embryos are opaque and cream-colored, and are comprised of an identifiable scutellum and coleoptile. The embryos are individually subcultured to a germination medium comprising MS salts and vitamins, 100 mg/l myo-inositol, 40 gm/l sucrose and 1.5 gm/l Gelrite in 100 X 25 mm Petri dishes and incubated under a 16 hour light:8 hour dark photoperiod and 40 meinsteinsm⁻²sec⁻¹ from cool-white fluorescent tubes. After about 7 days, the somatic embryos have germinated and produced a well-defined shoot and root. The individual plants are subcultured to germination medium in 125 X 25 mm glass tubes to allow further plant development. The plants are maintained under a 16 hour light:8 hour dark photoperiod and 40 meinsteinsm⁻²sec⁻¹ from cool-white fluorescent tubes. After about 7 days, the plants are well-established and are transplanted to horticultural soil, hardened off, and potted into commercial greenhouse soil mixture and grown to sexual maturity in a greenhouse. An elite inbred line is used as a male to pollinate regenerated transgenic plants.

### Agrobacterium-mediated:

When Agrobacterium-mediated transformation is used, the method of Zhao is employed as in PCT patent publication WO98/32326. Briefly, immature embryos are isolated from maize and the embryos contacted with a suspension of Agrobacterium (step 1: the infection step). In this step the immature embryos are preferably immersed in an Agrobacterium suspension for the initiation of inoculation. The embryos are co-cultured for a time with the Agrobacterium (step 2: the co-cultivation step). Preferably the immature embryos are cultured on solid medium following the infection step. Following this co-cultivation period an optional "resting" step is contemplated. In this resting step, the embryos are incubated in the presence of at least one antibiotic known to inhibit the growth of Agrobacterium without the addition of a selective agent for plant transformants (step 3: resting step). Preferably the immature embryos are cultured on solid medium with antibiotic, but without a selecting agent, for elimination of Agrobacterium and for a resting phase for the infected cells. Next, inoculated embryos are cultured on medium containing a selective agent and growing transformed callus is recovered (step 4: the selection step). Preferably, the immature embryos are cultured on solid medium with a selective agent resulting in the selective growth of transformed cells. The callus is then regenerated into plants (step 5: the regeneration step) and preferably calli grown on selective medium are cultured on solid medium to regenerate the plants.

### Example 3: Identification of High Cytokinin Transgenic Corn Lines

The resulting transformants are screened for elevated levels of cytokinin using a combination of direct measurements and in vivo correlates.

### INCREASED FREQUENCY OF SEED SET AND INCREASED NUMBER OF SEEDS

### (lpt2:ipt constructs):

Because yield is a combination of both frequency of seed set and number of seeds per ear, seeds exhibiting an increased level of cytokinin in the early stages of seed set and formation should have ears with a corresponding increase in seed set and numbers.

Lpt2::ipt transformants were initiated using GS3 embryos and Agrobacterium-mediated transformation (12425). Plantlets were regenerated in 2-3 months in 1998 and these plantlets (TO's) were transferred to the greenhouse after an additional 2-3 months. At anthesis, T0's were crossed with HG11 and at maturity the ears were harvested, shelled and the seed used for additional seed propagation (both backcrossing to HG11 and self-pollinating). The number of seeds per T0 event, and the number of events which set seed were compared to a number of other transgenic events with promoter:gene combinations other than ltp2:ipt. These are shown in Table 1.

**Table 1. Seed set average of T0 events of ltp:ipt gene compared to other genes in T0 plants grown under identical green house conditions in 1998 in Johnston, IA.**

| **Inventive polynucleotide** | **gene description** | **number T0's** | **%T0 w/seed** | **average # seeds** |
|---|---|---|---|---|
| 12425 | ltp2:ipt | 35 | **82.9** | **198** |
| 12384 | lignin | 92 | 22.8 | 145 |
| 12417 | carbohydrate | 40 | 55.0 | 156 |
| 12427 | maturity | 35 | 45.7 | 69 |
| 12428 | lignin | 29 | 75.9 | 174 |
| 12723 | lignin | 35 | 62.9 | 184 |
| 12724 | lignin | 35 | 45.7 | 161 |

Compared to % seed set and average # seeds per T0 plant, lpt2:ipt, had both the highest % of T0 plants which set seed and the highest numerical average # of seeds compared to six other transgenic combinations in T0 plants grown at the same time and under the same greenhouse conditions. These results indicate that expression of cytokinin in the aleurone layer of early seed development may increase yield by increasing both the percentage of plants that set seed, and the number of seeds set per ear.

Subsequent generations will be grown at different field locations to determine their seed set and seed number characteristics and seed yield compared to non-transgenic controls of the same genetic background. Cytokinin levels will also be measured on transgenic and non-transgenic kernels of similar genetic background.

### Cytokinin determinations:

Samples can be collected and analyzed as follows. At 2, 6 and 22 DAP, 50 to 100 seeds can be collected from two replications per event (each replication composed of two subsamples) and the pedicel removed. For the 2, 6, and 22 DAP samples, the remaining seed tissue can be placed directly into liquid nitrogen (tissue defined as "seed," composed primarily of pericarp, aleurone, endosperm and nucellus).

### Sequence Descriptions:

SEQ ID NO.: 1
   cytox1-2 (maize cytokinin oxidase)
SEQ ID NO.:2
   Amino Acid sequence of cytox1-2
SEQ ID NO.: 3
   Primer for isolation of ipt gene
SEQ ID NO.: 4
   Primer for isolation of ipt gene
SEQ ID NO.: 5
   Primer for isolation of cytokinin oxidase gene
SEQ ID NO.: 6
   Primer for isolation of cytokinin oxidase gene
SEQ ID NO.: 8
   ltp2: ipt: gzw64a term
SEQ ID NO.: 10
   ipt probe
SEQ ID NO.: 11
   ipt probe

## Claims

1. A method for improving yield in *Zea mays* plants comprising:
- stably introducing into cells of said plants a genetic construct capable of preferentially expressing a cytokinin modulating gene which encodes a cytokinin biosynthetic enzyme in one or more tissues of developing seed and related maternal tissues within the range of from 14 days before pollination to 25 days after pollination; and
- regenerating and recovering plants from said cells;
wherein said cytokinin modulating gene is isopentenyl transferase and wherein said genetic construct comprises an ltp2 promoter directing temporal and/or spatial gene expression in plant seed operably linked to the cytokinin modulating gene.

2. A method according to claim 1 wherein the introduction of said construct is carried out by electroporation, PEG poration, particle bombardment, silicon fibre delivery, microinjection or Agrobacterium-mediated transformation.

3. A method according to claim 2 wherein said process of introduction is particle bombardment or Agrobacterium-mediated transformation.

4. A method according to any one of claims 1 to 3, wherein said promoter directs endosperm-preferred expression.

5. A method according to any one of claims 1 to 4 further comprising propagating a plant thus obtained to give progeny plants whose cells comprise the construct defined in any one of claims 1 or 4.

6. A method according to claim 5 wherein said propagation comprises the production of an inbred line whose cells comprise the construct defined in any one of claims 1 or 4.

7. Use of a stably introduced genetic construct capable of preferentially expressing a cytokinin modulating gene which encodes a cytokinin biosynthetic enzyme in one or more tissues of developing seed and related maternal tissue within the range of from 14 days before pollination to 25 days after pollination, to improve yield in a *Zea mays* plant, wherein said cytokinin modulating gene is isopentenyl transferase and wherein said genetic construct comprises an ltp2 promoter directing temporal and/or spatial gene expression in plant seed operably linked to the cytokinin modulating gene.

8. Use according to claim 7, wherein said promoter directs endosperm-preferred expression.

9. A fertile transgenic *Zea mays* plant comprising a genetic construct stably integrated into the genome thereof, wherein said construct comprises an ltp2 promoter operably linked to the cytokinin-modulating gene isopentenyl transferase, wherein said promoter directs temporal and/or spatial expression in plant seed.

10. Seeds of the transgenic plant of claim 9, wherein said seeds contain the genetic construct comprising the ltp2 promoter operably linked to the cytokinin-modulating gene isopentenyl transferase.

## Patentansprüche

1. Verfahren zur Verbesserung des Ertrags bei *Zea-mays-*Pflanzen, umfassend:
- stabiles Einführen in Zellen der Pflanzen eines genetischen Konstrukts, das fähig ist, ein Cytokinin modulierendes Gen, welches ein Cytokinin-Biosyntheseenzym codiert, in einem Gewebe oder mehreren Geweben von sich entwickelnden Samen und verwandten maternalen Geweben innerhalb des Bereichs von 14 Tage vor Bestäubung bis 25 Tage nach Bestäubung bevorzugt zu exprimieren, und
- Regenerieren und Gewinnen von Pflanzen aus den Zellen; wobei das Cytokinin modulierende Gen Isopentenyltransferase ist und wobei das genetische Konstrukt einen ltp2-Promotor, der eine zeitliche und/oder räumliche Genexpression in Pflanzensamen steuert, funktionell verknüpft mit dem Cytokinin modulierenden Gen umfasst.

2. Verfahren gemäß Anspruch 1, wobei die Einführung des Konstrukts durch Elektroporation, PEG-Poration, Teilchenbeschuss, Siliziumfaserabgabe, Mikroinjektion oder Agrobacterium-vermittelte Transformation durchgeführt wird.

3. Verfahren gemäß Anspruch 2, wobei das Verfahren des Einführens Teilchenbeschuss oder Agrobacterium-vermittelte Transformation ist.

4. Verfahren gemäß einem der Ansprüche 1 bis 3, wobei der Promotor eine Endosperm-bevorzugte Expression steuert.

5. Verfahren gemäß einem der Ansprüche 1 bis 4, das außerdem Vermehren einer so erhaltenen Pflanze unter Erhalt von Nachkommenpflanzen, deren Zellen das Konstrukt, das in einem der Ansprüche 1 oder 4 definiert ist, umfassen, umfasst.

6. Verfahren gemäß Anspruch 5, wobei die Vermehrung die Produktion einer Inzuchtlinie umfasst, deren Zellen das Konstrukt, das in einem der Ansprüche 1 bis 4 definiert ist, umfassen.

7. Verwendung eines stabil eingeführten genetischen Konstrukts, das fähig ist, ein Cytokinin modulierendes Gen, das ein Cytokinin-Biosyntheseenzym codiert, in einem Gewebe oder mehreren Geweben von sich entwickelndem Samen und verwandtem maternalen Gewebe innerhalb eines Bereichs von 14 Tage vor Bestäubung bis 25 Tage nach Bestäubung bevorzugt zu exprimieren, um den Ertrag bei einer *Zea-mays*-Pflanze zu verbessern, wobei das Cytokinin modulierende Gen Isopentenyltransferase ist und wobei das genetische Konstrukt einen ltp2-Promotor, der die zeitliche und/oder räumliche Genexpression in einem Pflanzensamen steuert, funktionell verknüpft mit dem Cytokinin modulierenden Gen umfasst.

8. Verwendung gemäß Anspruch 7, wobei der Promotor eine Endosperm-bevorzugte Expression steuert.

9. Fertile transgene Zea-mays-Pflanze, die ein genetisches Konstrukt stabil in das Genom derselben integriert umfasst, wobei das Konstrukt einen ltp2-Promotor funktionell verknüpft mit dem Cytokinin modulierenden Gen Isopentenyltransferase umfasst, wobei der Promotor die zeitliche und/oder räumliche Expression in Pflanzensamen steuert.

10. Samen der transgenen Pflanze gemäß Anspruch 9, wobei die Samen das genetische Konstrukt enthalten, das den ltp2-Promotor funktionell verknüpft mit dem Cytokinin modulierenden Gen Isopentenyltransferase umfasst.

## Revendications

1. Procédé pour améliorer le rendement en plantes de *Zea mays,* comprenant :
- l'introduction stable, dans des cellules desdites plantes, d'une construction génétique capable d'exprimer préférentiellement un gène modulateur de cytokinine qui code une enzyme de biosynthèse de cytokinine dans un ou plusieurs tissus d'une semence en développement et des tissus maternels apparentés dans la gamme de 14 jours avant la pollinisation à 25 jours après la pollinisation ; et
- la régénération et la récupération des plantes à partir desdites cellules ;
dans lequel ledit gène modulateur de cytokinine est l'isopentényl transférase et dans lequel ladite construction génétique comprend un promoteur Itp2 dirigeant l'expression génique temporelle et/ou spatiale dans une semence de plante lié de façon opérationnelle au gène modulateur de cytokinine.

2. Procédé selon la revendication 1, dans lequel l'introduction de ladite construction est effectuée par électroporation, PEG poration, bombardement de particules, délivrance de fibres de silicium, micro-injection ou transformation médiée par *Agrobacterium.*

3. Procédé selon la revendication 2, dans lequel ledit procédé d'introduction est un bombardement de particules ou une transformation médiée par *Agrobacterium.*

4. Procédé selon l'une quelconque des revendications 1 à 3, dans lequel ledit promoteur dirige l'expression à préférence pour l'endosperme.

5. Procédé selon l'une quelconque des revendications 1 à 4, comprenant en outre la propagation d'une plante ainsi obtenue pour donner des descendants dont les cellules comprennent la construction définie dans l'une quelconque des revendications 1 ou 4.

6. Procédé selon la revendication 5, dans lequel ladite propagation comprend la production d'une lignée consanguine dont les cellules comprennent la construction définie dans l'une quelconque des revendications 1 ou 4.

7. Utilisation d'une construction génétique introduite de façon stable capable d'exprimer préférentiellement un gène modulateur de cytokinine qui code une enzyme de biosynthèse de cytokinine dans un ou plusieurs tissus d'une semence en développement et un tissu maternel apparenté dans la gamme de 14 jours avant la pollinisation à 25 jours après la pollinisation, pour améliorer le rendement en une plante de *Zea mays,* dans laquelle ledit gène modulateur de cytokinine est l'isopentényl transférase et dans laquelle ladite construction génétique comprend un promoteur Itp2 dirigeant l'expression génique temporelle et/ou spatiale dans une semence de plante lié de façon opérationnelle au gène modulateur de cytokinine.

8. Utilisation selon la revendication 7, dans laquelle ledit promoteur dirige l'expression à préférence pour l'endosperme.

9. Plante de *Zea mays* transgénique fertile comprenant une construction génétique intégrée de façon stable dans le génome de celle-ci, dans laquelle ladite construction comprend un promoteur Itp2 lié de façon opérationnelle au gène modulateur de cytokinine isopentényl transférase, dans laquelle ledit promoteur dirige l'expression temporelle et/ou spatiale dans une semence de plante.

10. Semences de la plante transgénique selon la revendication 9, lesdites semences contenant la construction génétique comprenant le promoteur Itp2 lié de façon opérationnelle au gène modulateur de cytokinine isopentényl transférase.


### SEQUENCE LISTING

<110> Pioneer Hi-Bred International, Inc.
<120> Regulated Expression of Genes in Plant Seeds
<130> 0803-PCT
<141> 2000-04-07
<150> US 60/129,844
   <151> 1999-04-16
<160> 12
<170> FastSEQ for Windows Version 3.0
<210> 1
   <211> 1608
   <212> DNA
   <213> Zea mays
<220>
   <221> CDS
   <222> (1)...(1605)
<400> 1
   atg gcg gtg gtt tat tac ctg ctg ctg gcc ggg ctg atc gcc tgc tct       48
   Met Ala Val Val Tyr Tyr Leu Leu Leu Ala Gly Leu Ile Ala Cys Ser
    1               5                  10                  15
   cat gca cta gcg gca ggc acg ctt gcg ctc gga gaa gat cgc ggc cgt       96
   His Ala Leu Ala Ala Gly Thr Leu Ala Leu Gly Glu Asp Arg Gly Arg
               20                  25                  30
   ccc tgg cca gcc ttc ctc gcc gcg ctg gcc ttg gac ggc aag ctc cgg      144
   Pro Trp Pro Ala Phe Leu Ala Ala Leu Ala Leu Asp Gly Lys Leu Arg
           35                  40                  45
   acc gac agc aac gcg acg gcg gcg gcc tcg acg gac ttc ggc aac atc      192
   Thr Asp Ser Asn Ala Thr Ala Ala Ala Ser Thr Asp Phe Gly Asn Ile
       50                  55                  60
   acg tcg gcg ctc ccg gcg gcg gtc cta tac ccg tcg tcc acg ggc gac      240
   Thr Ser Ala Leu Pro Ala Ala Val Leu Tyr Pro Ser Ser Thr Gly Asp
    65                  70                  75                  80
   ctg gtg gcg ctg ctg agc gcg gcc aac tcc acc ccg ggg tgg ccc tac      288
   Leu Val Ala Leu Leu Ser Ala Ala Asn Ser Thr Pro Gly Trp Pro Tyr
                   85                  90                  95
   acc atc gcg ttc cgc ggc cgc ggc cac tcc ctc atg ggc cag gcc ttc      336
   Thr Ile Ala Phe Arg Gly Arg Gly His Ser Leu Met Gly Gln Ala Phe
               100                 105                 110
   gcc ccc ggc ggg gtg gtc gtc aac atg gcg tcc ctg ggc gac gcc gcc      384
   Ala Pro Gly Gly Val Val Val Asn Met Ala Ser Leu Gly Asp Ala Ala
           115                 120                 125
   gcc gcc gcg ccg ccg cgc gtc aac gtg tcc gcg gac ggc cgc tac gtg      432
   Ala Ala Ala Pro Pro Arg Val Asn Val Ser Ala Asp Gly Arg Tyr Val
       130                 135                 140
   gac gcc ggc ggc gag cag gtg tgg atc gac gtg ctg cgc gcg tct ctg      480
   Asp Ala Gly Gly Glu Gln Val Trp Ile Asp Val Leu Arg Ala Ser Leu
   145                 150                 155                 160
   gcg cgc ggc gtg gcg ccg cgc tcc tgg acc gac tac ctc tac ctc acc      528
   Ala Arg Gly Val Ala Pro Arg Ser Trp Thr Asp Tyr Leu Tyr Leu Thr
                   165                 170                 175
   gtc ggc ggc acg ctg tcc aac gca ggc atc agc ggc cag gcg ttc cgc      576
   Val Gly Gly Thr Leu Ser Asn Ala Gly Ile Ser Gly Gln Ala Phe Arg
               180                 185                 190
   cac ggc cca cag ata tct aac gtg ctg gag atg gac gtt atc acc ggc      624
   His Gly Pro Gln Ile Ser Asn Val Leu Glu Met Asp Val Ile Thr Gly
           195                 200                 205
   cat ggg gag atg gtg acg tgc tcc aag cag ctg aac gcg gac ctg ttc      672
   His Gly Glu Met Val Thr Cys Ser Lys Gln Leu Asn Ala Asp Leu Phe
       210                 215                 220
   gac gcc gtc ctg ggc ggg ctg ggg cag ttc gga gtg atc acc cgg gcc      720
   Asp Ala Val Leu Gly Gly Leu Gly Gln Phe Gly Val Ile Thr Arg Ala
   225                 230                 235                 240
   cgg atc gcg gtg gag ccg gcg ccg gcg cgg gcg cgg tgg gtg cgg ctc      768
   Arg Ile Ala Val Glu Pro Ala Pro Ala Arg Ala Arg Trp Val Arg Leu
                   245                 250                 255
   gtg tac acc gac ttc gcg gcg ttc agc gcc gac cag gag cgg ctg acc      816
   Val Tyr Thr Asp Phe Ala Ala Phe Ser Ala Asp Gln Glu Arg Leu Thr
               260                 265                 270
   gcc ccg cgg ccc ggc ggc ggc ggc gcg tcg ttc ggc ccg atg agc tac      864
   Ala Pro Arg Pro Gly Gly Gly Gly Ala Ser Phe Gly Pro Met Ser Tyr
           275                 280                 285
   gtg gaa ggg tcg gtg ttc gtg aac cag agc ctg gcg acc gac ctg gcg      912
   Val Glu Gly Ser Val Phe Val Asn Gln Ser Leu Ala Thr Asp Leu Ala
       290                 295                 300
   aac acg ggg ttc ttc acc gac gcc gac gtc gcc cgg atc gtc gcg ctc      960
   Asn Thr Gly Phe Phe Thr Asp Ala Asp Val Ala Arg Ile Val Ala Leu
   305                 310                 315                 320
   gcc ggg gag cgg aac gcc acc acc gtg tac agc atc gag gcc acg ctc     1008
   Ala Gly Glu Arg Asn Ala Thr Thr Val Tyr Ser Ile Glu Ala Thr Leu
                   325                 330                 335
   aac tac gac aac gcc acg gcg gcg gcg gcg gtg gac cag gag ctc gcg     1056
   Asn Tyr Asp Asn Ala Thr Ala Ala Ala Ala Val Asp Gln Glu Leu Ala
               340                 345                 350
   tcc gtg ctg ggc acg ctg agc tac gtg gaa ggg ttc gcg ttc cag cgc     1104
   Ser Val Leu Gly Thr Leu Ser Tyr Val Glu Gly Phe Ala Phe Gln Arg
           355                 360                 365
   gac gtg tcc tac acg gcg ttc ctt gac cgg gtg cac ggc gag gag gtg 1152
   Asp Val Ser Tyr Thr Ala Phe Leu Asp Arg Val His Gly Glu Glu Val
   370 375 380
   gcg ctc aac aag ctg ggg ctg tgg cgg gtg ccg cac ccg tgg ctc aac 1200
   Ala Leu Asn Lys Leu Gly Leu Trp Arg Val Pro His Pro Trp Leu Asn
   385 390 395 400
   atg ttc gtg ccg cgc tcg cgc atc gcc gac ttc gac cgc ggc gtc ttc 1248
   Met Phe Val Pro Arg Ser Arg Ile Ala Asp Phe Asp Arg Gly Val Phe
   405 410 415
   aag ggc atc ttg cag ggc acc gac atc gtc ggc ccg ctc atc gtc tac 1296
   Lys Gly Ile Leu Gln Gly Thr Asp Ile Val Gly Pro Leu Ile Val Tyr
   420 425 430
   ccc ctc aac aaa tcc atg tgg gac gac ggc atg tcg gcg gcg acg ccg 1344
   Pro Leu Asn Lys Ser Met Trp Asp Asp Gly Met Ser Ala Ala Thr Pro
   435 440 445
   tcg gag gac gtg ttc tac gcg gtg tcg ctg ctc ttc tcg tcg gtg gcg 1392
   Ser Glu Asp Val Phe Tyr Ala Val Ser Leu Leu Phe Ser Ser Val Ala
   450 455 460
   ccc aac gac ctg gcg agg ctg cag gag cag aac agg agg atc ctg cgc 1440
   Pro Asn Asp Leu Ala Arg Leu Gln Glu Gln Asn Arg Arg Ile Leu Arg
   465 470 475 480
   ttc tgc gac ctc gcc ggg atc cag tac aag acc tac ctg gcg cgg cac 1488
   Phe Cys Asp Leu Ala Gly Ile Gln Tyr Lys Thr Tyr Leu Ala Arg His
   485 490 495
   acg gac cgc agt gac tgg gtc cgc cac ttc ggc gcc gcc gag tgg aat 1536
   Thr Asp Arg Ser Asp Trp Val Arg His Phe Gly Ala Ala Glu Trp Asn
   500 505 510
   cgc ttc gtg gag atg aag aac aag tac gac ccc aag agg ctg ctc tcc 1584
   Arg Phe Val Glu Met Lys Asn Lys Tyr Asp Pro Lys Arg Leu Leu Ser
   515 520 525
   ccc ggc cag gac atc ttc aac tga 1608
   Pro Gly Gln Asp Ile Phe Asn
   530 535
<210> 2
   <211> 535
   <212> PRT
   <213> Zea mays
<400> 2
   Met Ala Val Val Tyr Tyr Leu Leu Leu Ala Gly Leu Ile Ala Cys Ser
   1 5 10 15
   His Ala Leu Ala Ala Gly Thr Leu Ala Leu Gly Glu Asp Arg Gly Arg
   20 25 30
   Pro Trp Pro Ala Phe Leu Ala Ala Leu Ala Leu Asp Gly Lys Leu Arg
   35 40 45
   Thr Asp Ser Asn Ala Thr Ala Ala Ala Ser Thr Asp Phe Gly Asn Ile
   50 55 60
   Thr Ser Ala Leu Pro Ala Ala Val Leu Tyr Pro Ser Ser Thr Gly Asp
   65 70 75 80
   Leu Val Ala Leu Leu Ser Ala Ala Asn Ser Thr Pro Gly Trp Pro Tyr
                   85                  90                  95
   Thr Ile Ala Phe Arg Gly Arg Gly His Ser Leu Met Gly Gln Ala Phe
               100                 105                 110
   Ala Pro Gly Gly Val Val Val Asn Met Ala Ser Leu Gly Asp Ala Ala
           115                 120                 125
   Ala Ala Ala Pro Pro Arg Val Asn Val Ser Ala Asp Gly Arg Tyr Val
       130                 135                 140
   Asp Ala Gly Gly Glu Gln Val Trp Ile Asp Val Leu Arg Ala Ser Leu
   145                 150                 155                 160
   Ala Arg Gly Val Ala Pro Arg Ser Trp Thr Asp Tyr Leu Tyr Leu Thr
                   165                 170                 175
   Val Gly Gly Thr Leu Ser Asn Ala Gly Ile Ser Gly Gln Ala Phe Arg
               180                 185                 190
   His Gly Pro Gln Ile Ser Asn Val Leu Glu Met Asp Val Ile Thr Gly
           195                 200                 205
   His Gly Glu Met Val Thr Cys Ser Lys Gln Leu Asn Ala Asp Leu Phe
       210                 215                 220
   Asp Ala Val Leu Gly Gly Leu Gly Gln Phe Gly Val Ile Thr Arg Ala
   225                 230                 235                 240
   Arg Ile Ala Val Glu Pro Ala Pro Ala Arg Ala Arg Trp Val Arg Leu
                   245                 250                 255
   Val Tyr Thr Asp Phe Ala Ala Phe Ser Ala Asp Gln Glu Arg Leu Thr
               260                 265                 270
   Ala Pro Arg Pro Gly Gly Gly Gly Ala Ser Phe Gly Pro Met Ser Tyr
           275                 280                 285
   Val Glu Gly Ser Val Phe Val Asn Gln Ser Leu Ala Thr Asp Leu Ala
       290                 295                 300
   Asn Thr Gly Phe Phe Thr Asp Ala Asp Val Ala Arg Ile Val Ala Leu
   305                 310                 315                 320
   Ala Gly Glu Arg Asn Ala Thr Thr Val Tyr Ser Ile Glu Ala Thr Leu
                   325                 330                 335
   Asn Tyr Asp Asn Ala Thr Ala Ala Ala Ala Val Asp Gln Glu Leu Ala
               340                 345                 350
   Ser Val Leu Gly Thr Leu Ser Tyr Val Glu Gly Phe Ala Phe Gln Arg
           355                 360                 365
   Asp Val Ser Tyr Thr Ala Phe Leu Asp Arg Val His Gly Glu Glu Val
       370                 375                 380
   Ala Leu Asn Lys Leu Gly Leu Trp Arg Val Pro His Pro Trp Leu Asn
   385                 390                 395                 400
   Met Phe Val Pro Arg Ser Arg Ile Ala Asp Phe Asp Arg Gly Val Phe
                   405                 410                 415
   Lys Gly Ile Leu Gln Gly Thr Asp Ile Val Gly Pro Leu Ile Val Tyr
               420                 425                 430
   Pro Leu Asn Lys Ser Met Trp Asp Asp Gly Met Ser Ala Ala Thr Pro
           435                 440                 445
   Ser Glu Asp Val Phe Tyr Ala Val Ser Leu Leu Phe Ser Ser Val Ala
       450                 455                 460
   Pro Asn Asp Leu Ala Arg Leu Gln Glu Gln Asn Arg Arg Ile Leu Arg
   465                 470                 475                 480
   Phe Cys Asp Leu Ala Gly Ile Gln Tyr Lys Thr Tyr Leu Ala Arg His
                   485                 490                 495
   Thr Asp Arg Ser Asp Trp Val Arg His Phe Gly Ala Ala Glu Trp Asn
               500                 505                 510
   Arg Phe Val Glu Met Lys Asn Lys Tyr Asp Pro Lys Arg Leu Leu Ser
           515                 520                 525
   Pro Gly Gln Asp Ile Phe Asn
       530                 535
<210> 3
   <211> 51
   <212> DNA
   <213> Artificial Sequence
<220>
   <223> Synthesized based on sequence from Agrobacterium tumefaciens
<400> 3
   caucaucauc auggatccac caatggatct acgtctaatt ttcggtccaa c 51
<210> 4
   <211> 42
   <212> DNA
   <213> Artificial Sequence
<220>
   <223> Synthesized based on sequence from Agrobacterium tumefaciens
<400> 4
   cuacuacuac uagttaactc acattcgaaa tggtggtcct tc 42
<210> 5
   <211> 29
   <212> DNA
   <213> Zea mays
<400> 5
   catgccatgg cggtggttta ttacctgct 29
<210> 6
   <211> 31
   <212> DNA
   <213> Zea mays
<400> 6
   cgggatcctc atcatcagtt gaagatgtcc t 31
<210> 7
   <211> 0
   <212> DNA
   <213> Artificial Sequence
<400> 7
   000
<210> 8
   <211> 2722
   <212> DNA
   <213> Artificial Sequence
<220>
   <223> Promoter from Hordeum vulgare, Plant Journal 6:849-860 (1994); gene from Agrobacterium tumefaciens, Molecular and General Genetics 216:388-394 (1989); terminator from Zea mays, Genbank Accession #S78780.
<400> 8
   cggccgctct agaactagtg gatctcgatg tgtagtctac gagaagggtt aaccgtctct      60
   tcgtgagaat aaccgtggcc taaaaataag ccgatgagga taaataaaat gtggtggtac     120
   agtacttcaa gaggtttact catcaagagg atgcttttcc gatgagctct agtagtacat     180
   cggacctcac atacctccat tgtggtgaaa tattttgtgc tcatttagtg atgggtaaat     240
   tttgtttatg tcactctagg ttttgacatt tcagttttgc cactcttagg ttttgacaaa     300
   taatttccat tccgcggcaa aagcaaaaca attttatttt acttttacca ctcttagctt     360
   tcacaatgta tcacaaatgc cactctagaa attctgttta tgccacagaa tgtgaaaaaa     420
   aacactcact tatttgaagc caaggtgttc atggcatgga aatgtgacat aaagtaacgt     480
   tcgtgtataa gaaaaaattg tactcctcgt aacaagagac ggaaacatca tgagacaatc     540
   gcgtttggaa ggctttgcat cacctttgga tgatgcgcat gaatggagtc gtctgcttgc     600
   tagccttcgc ctaccgccca ctgagtccgg gcggcaacta ccatcggcga acgacccagc     660
   tgacctctac cgaccggact tgaatgcgct accttcgtca gcgacgatgg ccgcgtacgc     720
   tggcgacgtg cccccgcatg catggcggca catggcgagc tcagaccgtg cgtggctggc     780
   tacaaatacg taccccgtga gtgccctagc tagaaactta cacctgcaac tgcgagagcg     840
   agcgtgtgag tgtagccgag tagatccccc gggctgcagc ttatttttac aacaattacc     900
   aacaacaaca aacaacaaac aacattacaa ttactattta caattacagt cgacggatca     960
   agtgcaaagg tccgccttgt ttctcctctg tctcttgatc tgactaatct tggtttatga    1020
   ttcgttgagt aattttgggg aaagcttcgt ccacagtttt tttttcgatg aacagtgccg    1080
   cagtggcgct gatcttgtat gctatcctgc aatcgtggtg aacttatgtc ttttatatcc    1140
   ttcactacca tgaaaagact agtaatcttt ctcgatgtaa catcgtccag cactgctatt    1200
   accgtgtggt ccatccgaca gtctggctga acacatcata cgatattgag caaagatcga    1260
   tctatcttcc ctgttcttta atgaaagacg tcattttcat cagtatgatc taagaatgtt    1320
   gcaacttgca aggaggcgtt tctttctttg aatttaacta actcgttgag tggccctgtt    1380
   tctcggacgt aaggcctttg ctgctccaca catgtccatt cgaattttac cgtgtttagc    1440
   aagggcgaaa agtttgcatc ttgatgattt agcttgacta tgcgattgct ttcctggacc    1500
   cgtgcagctg cggacggatc caccatggat ctacgtctaa ttttcggtcc aacttgcaca    1560
   ggaaagacat cgactgcgat agctcttgcc cagcagactg gcctcccagt cctctcgctc    1620
   gatcgcgtcc aatgctgtcc tcaactatca accggaagcg ggcgaccaac agtggaagaa    1680
   ctgaaaggaa cgactcgtct gtaccttgat gatcgccctt tggtaaaggg tatcattaca    1740
   gccaagcaag ctcatgaacg gctcattgcg gaggtgcaca atcacgaggc caaaggcggg    1800
   cttattcttg agggaggatc tatctcgttg ctcaggtgca tggcgcaaag tcgttattgg    1860
   aacgcggatt ttcgttggca tattattcgc aacgagttag cagacgagga gagcttcatg    1920
   agcgtggcca agaccagagt taagcagatg ttacgcccct ctgcaggtct ttctattatc    1980
   caagagttgg ttcaactttg gagggagcct cggctgaggc ccatactgga agggatcgat    2040
   ggatatcgat atgccctgct atttgctacc cagaaccaga tcacgcccga tatgctattg    2100
   cagctcgacg cagatatgga gaataaattg attcacggta tcgctcagga gtttctaatc    2160
   catgcgcgtc gacaggaaca gaaattccct ttggtgggcg cgacagctgt cgaagcgttt    2220
   gaaggaccac catttcgaat gtgagttgat ccccggcggt gtcccccact gaagaaacta    2280
   tgtgctgtag tatagccgct ggctagctag ctagttgagt catttagcgg cgatgattga    2340
   gtaataatgt gtcacgcatc accatgcatg ggtggcagtc tcagtgtgag caatgacctg    2400
   aatgaacaat tgaaatgaaa agaaaaaagt attgttccaa attaaacgtt ttaacctttt    2460
   aataggttta tacaataatt gatatatgtt ttctgtatat gtctaatttg ttatcatcca    2520
   tttagatata gacgaaaaaa aatctaagaa ctaaaacaaa tgctaatttg aaatgaaggg    2580
   agtatatatt gggataatgt cgatgagatc cctcgtaata tcaccgacat cacacgtgtc    2640
   cagttaatgt atcagtgata cgtgtattca catttgttgc gcgtaggcgt acccaacaat    2700
   tttgatcgac tatcagaaag tc                                              2722
<210> 9
   <211> 0
   <212> DNA
   <213> Artificial Sequence
<400> 9
   000
<210> 10
   <211> 23
   <212> DNA
   <213> Artificial Sequence
<220>
   <223> Synthesized based on sequence from Agrobacterium tumefaciens
<400> 10
   gcgtccaatg ctgtcctcaa cta 23
<210> 11
   <211> 23
   <212> DNA
   <213> Artificial Sequence
<220>
   <223> Synthesized based on sequence from Agrobacterium tumefaciens
<400> 11
   gctctcctcg tctgctaact cgt 23
<210> 12
   <211> 0
   <212> DNA
   <213> Artificial Sequence
<400> 12
   000